# EUROPEAN PATENT SPECIFICATION

(11) **EP 0 859 831 B1**
(45) Date of publication and mention of the grant of the patent: **03.03.2010**
(21) Application number: 96932349.2
(22) Date of filing: 27.09.1996
(51) Int. Cl.: C12N 7/04, C07K 14/135

(54) **PRODUCTION OF INFECTIOUS RESPIRATORY SYNCYTIAL VIRUS FROM CLONED NUCLEOTIDE SEQUENCES**
HERSTELLUNG VON INFEKTIÖSER RESPIRATORISCHER SYNZITIAL VIRUS AUS KLONIERTEN NUKLEOTID SEQUENZEN
PRODUCTION DE VIRUS SYNCYTIAL RESPIRATOIRE INFECTIEUX A PARTIR DE SEQUENCES DE NUCLEOTIDES CLONES

(30) Priority: 27.09.1995 US 607083
(43) Date of publication of application: 26.08.1998
(73) Proprietor: THE UNITED STATES OF AMERICA, as represented by the Secretary of the Department of Health and Human Services, Bethesda, Maryland 20892 (US)
(72) Inventor: COLLINS, Peter, L., Rockville, MD 20852 (US)
(74) Representative: Campbell, Patrick John Henry
(86) International application number: PCT/US1996/015524
(87) International publication number: WO 1997/012032

(56) References cited:
- WO-A-92/01471
- WO-A-92/07940
- WO-A-93/21310
- WO-A-94/08022
- WO-A2-00/61611
- JOURNAL OF VIROLOGY, vol. 69, no. 9, September 1995, pages 5677-5686, XP000612069 GROSFELD H. ET AL.: "RNA replication by respiratory syncytial virus (RSV) is directed by the N, P, and L proteins; transcription also occurs under these conditions but requires RSV superinfection for efficient synthesis of full-lenght mRNA" cited in the application
- PROCEEDINGS OF THE NATIONAL ACADEMY OF SCIENCES OF USA, vol. 88, no. 21, November 1991, WASHINGTON US, pages 9663-9667, XP000196754 COLLINS P.L. ET AL.: "Rescue of syntethic analogs of respiratory syncytial virus genomic RNA and effect of truncations and mutations on the expression of a foreign reporter gene" cited in the application
- PROCEEDINGS OF THE NATIONAL ACADEMY OF SCIENCES OF USA, vol. 92, no. 25, 5 December 1995, WASHINGTON, US, pages 11563-11567, XP000196787 COLLINS P.L. ET AL.: "Production of infectious human respiratory syncytial virus from cloned cDNA confirms an essential role for the transcription elongation factor from the 5' proximal open reading frame of the M2 mRNA in gene expression and provides a capability for vaccine development" cited in the application
- EMBO JOURNAL, vol. 13, no. 18, 1994, EYNSHAM, OXFORD GB, pages 4195-4203, XP000612065 SCHNELL M.J. ET AL.: "Infectious rabies viruses from cloned cDNA" cited in the application
- COLLINS P.L. ET AL.: "The two open reading frames of the 22K mRNA of human respiratory syncytial virus: sequence comparison of antigenic subgroups A and B and expression in vitro" JOURNAL OF GENRAL VIROLOGY, vol. 71, no. 12, 1990, pages 3015-3020,
- ELANGO N. ET AL.: "mRNA sequence of three respiratory syncytial virus genes encoding to nonstructural proteins and a 22K structural protein" JOURNAL OF VIROLOGY, vol. 55, no. 1, 1985, pages 101-110,
- HACKING D. ET AL.: 'Respiratory Syncytial Virus-Viral Biology and the Host Response' JOURNAL OF INFECTION vol. 45, 2002, pages 18 - 24
- Retrieved from the Internet: <URL:http//www.who.int./vaccine_research/di seases/ari/en/index3.html>
- Retrieved from the Internet: <URL:http//www.immunizationinfo.org/vaccine _development_detail.cfv?id=100>
- Retrieved from the Internet: <URL:http//www.vaccinecheck,.com/vacinfoPri nt4-3.jsp>
- COLLINS J.E. ET AL.: 'Respiratory Syncytial Virus Infections in Children and Adults' JOURNAL OF INFECTION vol. 45, 2002, pages 10 - 17
- CROWE J.E.: "Respiratory syncytial virus vaccine development" VACCINE, vol. 20, 2002, pages S32-S37,
- MURPHY B.R. ET AL.: "An update on approaches to the development of respiratory syncytial virus (RSV) and parainfluenza virus type 3 (PIV3) vaccines" VIRUS RESEARCH, vol. 32, 1994, pages 13-36,
- CROWE J.E.: "Current approaches to the development of vaccines against disease caused by respiratory syncytial virus (RSV) and parainfluenza virus (PIV)" VACCINE, vol. 13, no. 4, 1995, pages 415-421,
- WHITEHEAD S. S. ET AL.: "Recombinant Respiratory Syncytial Bearing a Deletion of either the NS2 or SH Gene Is Attenuated in Chimpanzees" JOURNAL OF VIROLOGY, vol. 73, no. 4, 1999, pages 3438-3442,
- HARKER J. ET AL.: "Virally Delivered Cytokines Alter the Immune Response to Future Lung Infections" JOURNAL OF VIROLOGY, vol. 81, no. 23, 2007, pages 13105-13111, XP009093388
- WHITEHEAD S.S.: "Addition of a Missense Mutation Present in the L Gene of Respiratory Syncytial Virus(RSV) cpts 530/1032 to RSV Vaccine Candidate cpts248/404 Increases Its Attenuation and Temperature Sensitivity" JOURNAL OF VIROLOGY, vol. 73, no. 2, 1999, pages 871-877,
- KOBS G.: "In vitro Transcription from Biotinylated DNA Immobilized on Streptavidin MagneSpere Paramagnetic Particles" PROMEGA NOTES MAGAZINE, no. 60, 1996, page 20,

## Description

### Background of the Invention

Human respiratory syncytial virus (RSV) is the most important pediatric respiratory pathogen worldwide. This ubiquitous, highly infectious agent emerges each year in seasonal epidemics. Nearly everyone is infected at least once within the first two years of life. RSV disease is responsible for considerable morbidity and mortality, especially in the very young; in the United States it causes an estimated 91,000 hospitalizations and 4500 deaths annually, and its impact is much greater in less affluent countries. RSV also has come to be recognized as an important agent of disease of immunocompromised adults and of the elderly.

Resistance to RSV reinfection induced by natural infection is incomplete but increases incrementally with repeated exposure. Thus, RSV can infect multiple times during childhood and life, but serious disease usually is limited to the first and sometimes second infections of life. The minimum goal of RSV immunoprophylaxis is to induce sufficient resistance to prevent serious disease associated with the initial infections.

A number of attenuated RSV strains were developed and evaluated as vaccines during the 1960's and 70's, but they were found to be either over- or under-attenuated, and in some cases exhibited genetic instability, as is common for single-stranded RNA viruses. Current strategies under investigation for RSV vaccine development are principally the parenteral administration of purified viral antigen or the development of live attenuated RSV for intranasal administration. The intranasal route provides direct stimulation of local immunity. It also partially abrogates the immunosuppressive effects of RSV-specific maternally derived serum antibodies, which typically are found in the very young. The parenteral administration of inactivated RSV or purified RSV antigen in experimental animals appears to be associated with enhanced immunopathology upon subsequent virus challenge, similar to the enhanced RSV disease associated with a formalin-inactivated vaccine evaluated in the 1960's. But this effect has never been observed with RSV infection of the respiratory tract, suggesting that live attenuated viruses have an important advantage in safety. To date, however, there is no approved vaccine or highly effective antiviral therapy for RSV.

Research efforts to produce a suitable RSV vaccine are impeded by poor viral growth in tissue culture, a lengthy replication cycle, virion instability, a negative-sense RNA genome, and a complex genome organization and gene products. RSV is a member of the pneumovirus genus of the paramyxovirus family, and its genome of single-stranded negative-sense RNA of 15,222 nucleotides has been sequenced completely for wild-type strain A2 virus as well as for an attenuated derivative thereof.

Some aspects of RNA synthesis by RSV appear to follow the general pattern of nonsegmented negative strand viruses. The genome template is tightly encapsidated with the major nucleocapsid (N) protein and is associated with the phosphoprotein (P) and large (L) polymerase subunit protein. Transcription begins at the 3' extragenic leader region and proceeds along the entire length by a sequential, stop-start mechanism guided by short template signals flanking the genes. This yields at least ten major species of mRNA which encode at least ten major proteins. RNA replication occurs by a switch to the synthesis of a full length positive-sense "antigenome" which also is tightly encapsidated and serves as the template for the synthesis of progeny genome.

The viral genomic RNA of negative-strand viruses is not infectious alone as free RNA. In virions or intracellularly, viral RNA is always found tightly encapsidated in a ribonucleoprotein core. This nucleocapsid contains the viral proteins necessary for transcription and replication and has long been regarded as the minimum unit of infectivity (Brown et al., J. Virol. 1: 368-373 (1967)). Thus, it has been recognized that the generation of biologically active synthetic viral RNA from cDNA will require complementation by viral protein, leading to the assembly of functional nucleocapsids (Collins et al., Proc. Natl. Acad. Sci. USA 88: 9663-9667 (1991), and Collins et al., Virology 195: 252-256 (1993)). The ability to produce live RSV from cDNA is of particular importance because it would permit the introduction of specific engineered changes, including attenuating mutations, into the genome of infectious virus in an effort to produce safe and effective RSV vaccines.

Short, internally-deleted analogs of genome or antigenome RNA ("minigenomes") have been shown to participate in transcription and replication when synthesized intracellularly in the presence of the appropriate viral proteins. For two rhabdoviruses, rabies and vesicular stomatitis viruses, infectious virus has been produced by coexpression of a complete cDNA-encoded antigenome RNA in the presence of the N, P and L proteins (Schnell et al., EMBO J. 13: 4195-4203 (1994) and Lawson et al., Proc. Natl. Acad. Sci. USA 92: 4477-4481 (1995)).

RSV possesses a number of properties which distinguishes it and other members of the genus *Pneumovirus* from the better characterized paramyxoviruses of the genera *Paramyxovirus, Rubulavirus* and *Morbillivirus.* These differences include a greater number of mRNAs, an unusual gene order at the 3' end of the genome, species-to-species variability in the order of the glycoprotein and M2 genes, a greater diversity in intergenic regions, an attachment protein that exhibits mucin-like characteristics, extensive strain-to-strain sequence diversity, and several proteins not found in any or most of the other nonsegmented negative strand RNA viruses.

RSV remains the most common cause of severe viral lower respiratory tract disease in infants and children. Consequently, an urgent need remains for the ability to engineer a safe and effective vaccine that is able to prevent the serious illness in this population that often requires hospitalization. Quite surprisingly, the present invention fulfills this and other related needs by providing methods for introducing defined, predetermined changes into infectious RSV.

WO 92/07940 (Samal) relates to genes that are derived from bovine respiratory syncytial virus (BRSV).

WO 94/08022 (CSIRO) relates to a vector for delivering a foreign gene to a target cell, for expression of the foreign gene.

Grosfeld et al, Journal of Virology, Vol. 69 No.9, September 1995 (pages 5677-5686) refers to RNA replication by a respiratory syncytial virus (RSV) being directed by the N, P & L proteins.

Collins et al, PNAS Volume 88, November 1991 (pages 9663-9667) relates to the rescue of synthetic analogs of RSV genomic RNA.

Schnell et al, The Embo Journal, Vol. 13, No. 8, pages 4195-4203, 1994, refers to infectious rabies viruses from cloned cDNA.

Murphy et al, Virus Research 32 (1994) 13-36 provides an update on approaches to the development of RSV and other vaccines.

James E. Crowe, Vaccine, Vol. 13, No.4, pages 415 - 421 (1995) refers to approaches to the development of vaccines against disease caused by RSV and paraifluenza virus (PIV).

### Summary of the Invention

The present invention provides a method for producing an infectious RSV from one or more polynucleotide molecules encoding said RSV, comprising:
coexpressing in a cell or cell-free lysate an expression vector which comprises a polynucleotide molecule encoding a RSV genome or antigenome, wherein the recombinant RSV genome or antigenome is recombinantly modified by the introduction of a mutation by insertion, rearrangement, deletion or substitution of one or more nucleotides, said mutation producing a phenotype of attenuation, temperature sensitivity, tissue culture adaption, host range restriction or an enhanced immunogenicity of the RSV particle compared to wild-type RSV virus, and an expression vector which comprises one or more polynucleotide molecules that encodes N, P and L proteins and an RNA polymerase elongation factor which is an M2 (ORF1) protein of RSV, thereby producing an infectious RSV particle.

The isolated infectious RSV particle can be a viral or sub viral particle. The isolated infectious RSV virus may be a human RSV, a bovine or murine RSV, or the genome or antigenome can be a chimera of two or more different RSV genomes, such as having nucleotide segments from human and bovine RSV.

In other embodiments the invention provides a system for recombinant production, a system for recombinant production of respiratory syncytial virus (RSV) particles comprising one or more expression vectors that comprise a polynucleotide encoding a RSV genome or antigenome, a polynucleotide encoding a nucleocapsid (N) protein, a polynucleotide encoding a nucleocapsid phosphoprotein (P), a polynucleotide encoding a large polymerase protein (L) and a polynucleotide encoding a RNA polymerase elongation factor protein that is an M2(ORF1) protein of RSV, in which upon expression of said N, P, L and M2 ORF1 proteins, an infectious, self-replicating RSV particle comprising a polynucleotide encoding a RSV genome or antigenome, and said N, P, L and M2 (ORF1) proteins is produced.

This can involve an expression vector which comprises an isolated polynucleotide molecule encoding an RSV genome or antigenome, wherein the recombinant RSV genome or antigenome is recombinantly modified by the introduction of a mutation by insertion, rearrangement, deletion or substitution of one or more nucleotides, said mutation producing a phenotype of attenuation, temperature sensitivity, cold-adaption, small plaque size, host range restriction or a change in an immunogenic of the RSV particle compared to wild-type RSV virus and an expression vector which comprised on or more isolated polynucleotide molecule that encodes N, P, L and RNA polymerase elongation factor proteins are coexpressed in a cell or cell-free lysate, thereby producing an infectious RSV parcel. The RSV genome or antigenome and the N, P, L and RNA polymerase elongation factor proteins can be coexpressed by the same or different expression vectors. In some instances the N, P, L and RNA polymerase elongation factor proteins are each encoded on different expression vectors. The polynucleotide molecule encoding the RSV genome or antigenome is from a human, bovine or murine RSV sequence, and can be a chimera of a human RSV strain sequence and at least one non-human RSV sequence, or can encodes the genome or antigenome of a wild-type RSV strain. The RSV genome or antigenome can be modified from a wild-type RSV strain by a nucleotide insertion, rearrangement, deletion or substitution, so as to encode a phenotypic alteration that results in attenuation, temperature-sensitivity, cold-adaptation, small plaque size, host range restriction, or change in an immunogenic epitope of RSV. The polynucleotide can encode a genome or antigenome of a non-human RSV virsus, or can be a chimera of a non-human RSV and at least one other RSV or human or non-human origin. The polynucleotide molecule encoding the genome or antigenome can also be modified to include a nucleotide sequence that encodes a cytokine, T-helper epitope, a G protein of a different RSV subgroup, a restriction site marker, or a protein of a microbial pathogen (e.g., virus, bacterium or fungus) capable of eliciting a protective immune response in the intended host.

In other embodiments the invention provides a cell or cell-free lysate, containing:
(a) an expression vector which comprises a polynucleotide molecule encoding a RSV genome or antigenome; and
(b) an expression vector which comprises one or more polynucleotide molecules that encodes N, P and L proteins and an RNA polymerase elongation factor which is an M2 (ORF1) protein of RSV;
whereby upon expression said RSV genome or antigenome and N, P, L and RNA polymerase elongation factor proteins combine to produce an infectious RSV particle;
and wherein the recombinant RSV genome or antigenome is recombinantly modified by the introduction of a mutation by insertion, rearrangement, deletion or substitution of one or more nucleotides, said mutation producing a phenotype of attenuation, temperature sensitivity, tissue culture adaption, host range restriction or an enhanced immunogenicity of the RSV particle compared to wild-type RSV virus.

Upon expression the genome or antigenome and N, P, L and RNA polymerase elongation factor proteins combine to produce an infectious RSV particle, such as viral or subviral particle.

In another aspect the invention provides a system for recombinant production of respiratory syncytial virus (RSV) particles comprising one or more DNA polynucleotide molecules, the molecules comprising a polynucleotide encoding a RSV genome or antigenome, a polynucleotide encoding a nucleocapsid (N) protein, a polynucleotide encoding nucleocapsid phosphorprotein (P), a polynucleotide encoding a large polymerase protein (L) and a polynucleotide encoding a RNA polymerase elongation factor protein that is an M2(ORF1) protein or RSV, in which upon expression of said N, P, L and M2 ORF1 proteins, an infectious, self-replicating RSV particle comprising a polynucleotide encoding a RSV genome or antigenome, and said N, P, L and M2 (ORF1) proteins is produced.

The RSV genome or antigenome can be a human RSV. sequence and modified versions thereof, such as that exemplified in SEQ ID NO:1 (which depicts the 5' to 3' positive-sense sequence whereas the genome itself is negative-sense). The polynucleotide can also encode a genome or antigenome of a nonhuman RSV virus, or encode a chimera of a nonhuman RSV and at least one other RSV of human or nonhuman origin.

### Brief Description of the Drawings

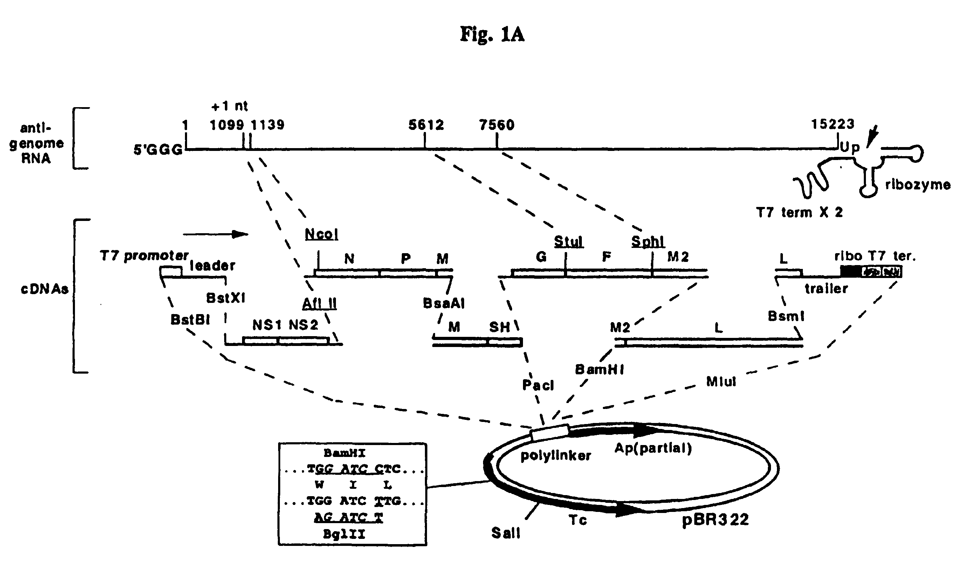

Fig, 1A and 1B show the construction of cDNA encoding RSV antigenome RNA, where Fig. 1A shows the structures of the cDNA and the encoded antigenome RNA (not to scale). The diagram of the antigenome includes the following features: the 5'-terminal nonviral G triplet contributed by the T7 promoter, the four sequence markers at positions 1099 '(which adds one nt to the length), 1139, 5611, and.7559 (numbering referring to the first base of the new restriction site), the ribozyme and tandem T7 terminators, and the single nonviral 3'-phosphorylated U residue contributed to the 3' end by ribozyme cleavage (the site of cleavage is indicated with an arrow). Cloned cDNA segments representing in aggregate the complete antigenome are also shown. The box illustrates the removal of the BamHI site, a modification that facilitated assembly: the naturally occurring BamHI-SalI fragment (the BamHI site is shown in the top line in positive sense, underlined) was replaced with a PCR-generated BglII-SalI fragment (the BglII site is shown in the bottom line, underlined; its 4-nt sticky end [shown in italics] is compatible with that of BamHI). This resulted in a single nt change (middle line, underlined) which was silent at the amino acid level.

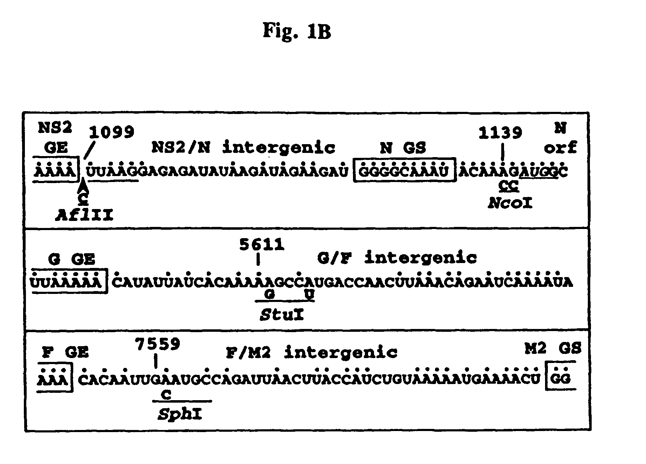

Fig. 1B shows the sequence markers contained in the cDNA-encoded antigenome RNA, where sequences are positive sense and numbered relative to the first nt of the leader region complement as 1; identities between strains A2 and 18537, representing subgroups A and B, respectively, are indicated with dots; sequences representing restriction sites in the cDNA are underlined; gene-start (GS) and gene-end (GE) transcription signals are boxed; the initiation codon of the N translational open reading frame at position 1141 is italicized, and the sequence markers are shown underneath each sequence. In the top sequence, a single C residue was inserted at position 1099 to create an AflII site in the NSII-N intergenic region, and the AG at positions 1139 and 1140 immediately upstream of the N translational open reading frame were replaced with CC to create a new NcoI site. In the middle sequence, substitution of G and U at positions 5612 and 5616, respectively, created a new StuI site in the G-F intergenic region. In the bottom sequence, a C replacement at position 7560 created a new SphI site in the F-M2 intergenic region.

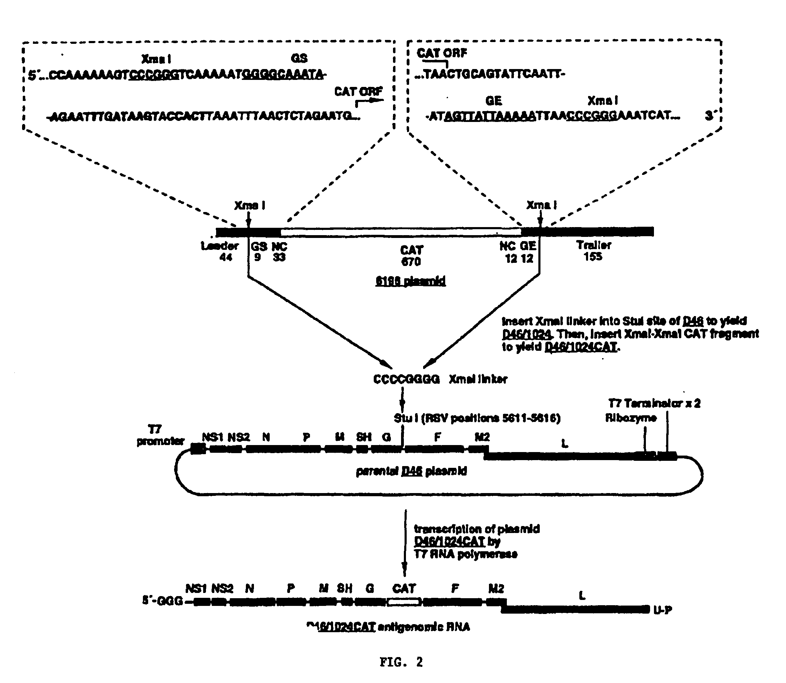

Fig. 2 shows construction of D46/1024CAT cDNA encoding an RSV antigenome containing the CAT ORF flanked by RSV transcription signals (not to scale, RSV-specific segments are shown as filled boxes and CAT sequence as an open box). The source of the CAT gene transcription cassette was RSV-CAT minigenome cDNA 6196 (diagram at top). The RSV-CAT minigenome contains the leader region, gene-start (GS) and gene-end (GE) signals, noncoding (NC) RSV gene sequences, and the CAT ORF, with *XmaI* restriction endonuclease sites preceding the GS signal and following the GE signal. The nucleotide lengths of these elements are indicated, and the sequences (positive-sense) surrounding the *XmaI* sites are shown above the diagram. A 8-nucleotide *XmaI* linker was inserted into *StuI* site of the parental plasmid D46 to construct the plasmid D46/1024. The *XmaI* - *XmaI* fragment of the plasmid 6196 was inserted into the plasmid D46/1024 to construct the plasmid D46/1024CAT. The RNA encoded by the D46 cDNA is shown at the bottom, including the three 5'-terminal nonviral G residues contributed by the T7 promoter and the 3'-terminal phosphorylated U residue contributed by cleavage of the hammerhead ribozyme; the nucleotide lengths do not include these nonviral nucleotides. The L gene is drawn offset to indicate the gene overlap.

### Description of the Specific Embodiments

The present invention provides the production of infectious RSV from cDNA. Infectious RSV is produced by the intracellular coexpression of a cDNA that encodes the RSV genome or antigenome RNA, together with those viral proteins necessary to generate a transcribing, replicating nucleocapsid, preferably one or more sequences that encode major nucleocapsid (N or NP) protein, nucleocapsid phosphoprotein (P), large (L) polymerase protein, and an M2(ORF1) protein. Infectious RSV particles are produced by the recombinant system. The recombinant production system permits the introduction of defined changes into infectious RSV, which changes are useful in a wide variety of applications such as: the development of live attenuated vaccine strains bearing predetermined, defined attenuating mutations; analysis of RSV molecular biology and pathogenesis using, e.g., defined mutations to alter functions or expression of RSV proteins; improvement in the growth in culture; identification of attenuating mutations in existing or future vaccine strains by distinguishing between silent incidental mutations versus those responsible for phenotype differences; production of modified vaccine virus to accommodate antigenic drift; enhancement of vaccine immunogenicity; ablation of epitopes associated with undesirable immunopathology; insertion of foreign genes, in whole or in part, encoding protective antigens to generate RSV strains capable of inducing immunity to both RSV and the virus or agent from which the protective antigen was derived; insertion of foreign genes, in whole or in part, encoding modulators of the immune system such as cytokines or T cell epitopes, to enhance the immunogenicity of the vaccine virus; etc.

According to the present invention cDNA encoding a RSV genome or antigenome is constructed for intracellular or in vitro coexpression with the necessary viral proteins to form infectious RSV. By "RSV antigenome" is meant an isolated positive-sense polynucleotide molecule which serves as the template for the synthesis of progeny RSV genome. Preferably a cDNA is constructed which is a positive-sense version of the RSV genome, corresponding to the replicative intermediate RNA, or antigenome, so as to minimize the possibility of hybridizing with positive-sense transcripts of the complementing sequences that encode proteins necessary to generate a transcribing, replicating nucleocapsid, i.e., sequences that encode N, P, L and M2(ORF1) protein. In an RSV minigenome system, genome and antigenome were equally active in rescue, whether complemented by RSV or by plasmids, indicating that either genome or antigenome can be used and thus the choice can be made on methodologic or other grounds.

A native RSV genome typically comprises a negative-sense polynucleotide molecule which, through complementary viral mRNAs, encodes eleven species of viral proteins, i.e., the nonstructural species NS1 and NS2, N, P, matrix (M), small hydrophobic (SH), glycoprotein (G), fusion (F), M2 (ORF1), M2(ORF2), and L, substantially as described in Mink et al., Virology 185: 615-624 (1991), Stec et al., Virology 183: 273-287 (1991), and Connors et al., Virol. 208:478-484 (1995). For purposes of the present invention the genome or antigenome of the recombinant RSV of the invention need only contain those genes or portions thereof necessary to render the viral or subviral particles encoded thereby infectious. Further, the genes or portions thereof may be provided by more than one polynucleotide molecule, i.e., a gene may be provided by complementation or the like from a separate nucleotide molecule.

By recombinant RSV is meant a RSV or RSV-like viral or subviral particle derived directly or indirectly from a recombinant expression system or propagated from virus or subviral particles produced therefrom. The recombinant expression system will employ a recombinant expression vector which comprises an operably linked transcriptional unit comprising an assembly of at least a genetic element or elements having a regulatory role in RSV gene expression, for example, a promoter, a structural or coding sequence which is transcribed into RSV RNA, and appropriate transcription initiation and termination sequences.

To produce infectious RSV from cDNA-expressed genome or antigenome, the genome or antigenome is coexpressed with those RSV proteins necessary to (i) produce a nucleocapsid capable of RNA replication, and (ii) render progeny nucleocapsids competent for both RNA replication and transcription. Transcription by the genome nucleocapsid provides the other RSV proteins and initiates a productive infection. Alternatively, additional RSV proteins needed for a productive infection can be supplied by coexpression.

An RSV antigenome may be constructed for use in the present invention by, e.g., assembling cloned cDNA segments, representing in aggregate the complete antigenome, by polymerase chain reaction (PCR; described in, e.g., U.S. Patent Nos. 4,683,195 and 4,683,202, and PCR Protocols: A Guide to Methods and Applications, Innis et al.; eds., Academic Press, San Diego (1990)) of reverse-transcribed copies of RSV mRNA or genome RNA. For example, cDNAs containing the lefthand end of the antigenome, spanning from an appropriate promoter (e.g., T7 RNA polymerase promoter) and the leader region complement to the SH gene, are assembled in an appropriate expression vector, such as a plasmid (e.g., pBR322) or various available cosmid, phage, or DNA virus vectors. The vector may be modified by mutagenesis and/or insertion of synthetic polylinker containing unique restriction sites designed to facilitate assembly. For example, a plasmid vector described herein was derived from pBR322 by replacement of the pstI-EcoR1 fragment with a synthetic DNA containing convenient restriction enzyme sites. pBR322 stabilized nucleotides 3716-3732 of the RSV sequence, which otherwise sustained nucleotide deletions or insertions, and propagation of the plasmid was in bacterial strain DH10B to avoid an artifactual duplication and insertion which otherwise occurred in the vicinity of nt4499. For ease of preparation the G, F and M2 genes can be assembled in a separate vector, as are the L and trailer sequences. The righthand end (e.g., L and trailer sequences) of the antigenome plasmid may contain additional sequences as desired, such as a flanking ribozyme and tandem T7 transcriptional terminators. The ribozyme can be hammerhead type (e.g., Grosfeld et al., J. Virol. 69:5677-5686 (1995)), which would yield a 3' end containing a single nonviral nucleotide, or can any of the other suitable ribozymes such as that of hepatitis delta virus (Perrotta et al., Nature 350:434-436 (1991)) which would yield a 3' end free of non-RSV nucleotides. A middle segment (e.g., G-to-M2 piece) is inserted into an appropriate restriction site of the leader-to-SH plasmid, which in turn is the recipient for the L-trailer-ribozyme-terminator piece, yielding a complete antigenome. In an illustrative example shown in Fig. 1A, the leader end was constructed to abut the promoter for T7 RNA polymerase which included three transcribed G residues for optimal activity; transcription donates these three nonviral G's to the 5' end of the antigenome. These three nonviral G residues can be omitted to yield a 5' end free of nonviral nucleotides. To generate a nearly-correct 3' end, the trailer end was constructed to be adjacent to a hammerhead ribozyme, which upon cleavage would donate a single 3'-phosphorylated U residue to the 3' end of the encoded RNA.

A variety of nucleotide insertions and deletions can be made in the RSV genome or antigenome. The nucleotide length of the genome of wild type human RSV (15,222 nucleotides) is a multiple of six, and members of the Paramyxovirus and Morbillivirus genera typically abide by a "rule of six," i.e., genomes (or minigenomes) replicate efficiently only when their nucleotide length is a multiple of six (thought to be a requirement for precise spacing of nucleotide residues relative to encapsidating NP protein). Alteration of RSV genome length by single residue increments had no effect on the efficiency of replication, and sequence analysis of several different minigenome mutants following passage showed that the length differences were maintained without compensatory changes. Thus, RSV lacks the strict requirement of genome length being a multiple of six, and nucleotide insertions and deletions can be made in the RSV genome or antigenome without defeating replication of the recombinant RSV of the present invention.

Alternative means to construct cDNA encoding the genome or antigenome include by reverse transcription-PCR using improved PCR conditions (e.g., as described in Cheng et al., Proc. Natl. Acad. Sci. USA 91:5695-5699 (1994)) to reduce the number of subunit cDNA components to as few as one or two pieces. In other embodiments different promoters can be used (e.g., T3, SP6) or different ribozymes (e.g., that of hepatitis delta virus. Different DNA vectors (e.g., cosmids) can be used for propagation to better accommodate the larger size genome or antigenome.

By virtue of the present invention a variety of alterations in the RSV genome or antigenome for incorporation into infectious recombinant RSV are made possible. For example, foreign genes may be inserted, the order of genes changed, gene overlap removed, the RSV gnome promoter replaced with its antigenome counterpart, portions of genes removed (e.g., the cytoplasmic tails of glycoprotein genes), and even entire genes deleted. Modifications in the sequence can be made to facilitate manipulations, such as the insertion of unique restriction sites in various intergenic regions (e.g., a unique Stul site between the G and F genes) or elsewhere. Nontranslated gene sequences can be removed to increase capacity for inserting foreign sequences.

The infectious RSV produced from cDNA-expressed genome or antigenome can be any of the RSV or RSV-like strains, e.g., human, bovine, murine, etc., or of any pneumovirus, e.g., pneumonia virus of mice or turkey rhinotracheitis virus. To engender a protective immune response, the RSV strain may be one which is endogenous to the subject being immunized, such as human RSV being used to immunize humans. The genome or antigenome can be modified, however, to express RSV genes from different types. Thus, infectious RSV intended for administration to humans may be human RSV that has been modified to contain genes from a bovine or murine RSV type such as for the purpose of attenuation, or a bovine RSV may be modified to contain genes that encode epitopes or proteins that elicit protection against human RSV infection, e.g., the human RSV glycoprotein genes can be substituted for the bovine glycoprotein genes such that the bovine RSV, which has a restricted ability to replicate in a human host, elicits a protective immune response in humans against human RSV strains.

The N, P and L proteins, necessary for RNA replication, require an RNA polymerase elongation factor such as the M2(ORF1) protein for processive transcription. Thus M2(ORF1) or a substantially equivalent transcription elongation factor for negative strand RNA viruses is required for the production of infectious RSV and is a necessary component of functional nucleocapsids during productive infection. The need for the M2(ORF1) protein is consistent with its role as a transcription elongation factor. The need for expression of the RNA polymerase elongation factor protein for negative strand RNA viruses is a feature of the present invention. M2(ORF1) can be supplied by expression of the complete M2-gene, although in this form the second ORF2 may also be expressed and have an inhibitory effect on RNA replication. Therefore, for production of infectious virus using the complete M2 gene the activities of the two ORFs should be balanced to permit sufficient expression of M(ORF1) to provide transcription elongation activity yet not so much of M(ORF2) to inhibit RNA replication. Alternatively, the ORF1 protein is provided from a cDNA engineered to lack ORF2 or which encodes a defective ORF2. Efficiency of virus production may also be improved by co-expression of additional viral protein genes, such as those encoding envelope constituents (i.e., SH, M, G, F proteins).

Isolated polynucleotides (e.g., cDNA) encoding the genome or antigenome and, separately, the N, p, L and M2(ORF1) proteins, are inserted by transfection, electroporation, mechanical insertion, transduction or the like, into cells which are capable of supporting a productive RSV infection, e.g., HEp-2, FRhL-DBS2, MRC, and Vero cells. Transfection of isolated polynucleotide sequences may be introduced into cultured cells by, for example, calcium phosphate-mediated transfection (Wigler et al., Cell 14: 725 (1978); Corsaro and Pearson, Somatic Cell Genetics 7: 603 (1981); Graham and Van der Eb, Virology 52: 456 (1973)), electroporation (Neumann et al., EMBO J. 1: 841-845 (1982)), DEAE-dextran mediated transfection (Ausubel et al., (ed.) Current Protocols in Molecular Biology, John Wiley and Sons, Inc., NY (1987)), cationic lipid-mediated transfection (Hawley-Nelson et al., Focus 15: 73-79 (1993)) or a commercially available transfection regent, e.g., LipofectACE® (Life Technologies). The N, P, L and M2(ORF1) proteins are encoded by one or more expression vectors which can be the same or separate from that which encodes the genome or antigenome, and various combinations thereof. Additional proteins may be included as desired, encoded on a its own vector or on a vector encoding a N, P, L, or M2(ORF1) protein or the complete genome or antigenome. Expression of the genome or antigenome and proteins from transfected plasmids can be achieved, for example, by each cDNA being under the control of a promoter for T7 RNA polymerase, which in turn is supplied by infection, transfection or transduction with an expression system for the T7 RNA polymerase, e.g., a vaccinia virus MVA strain recombinant which expresses the T7 RNA polymerase (Wyatt et al., Virology, 210 : 202-205 (1995)). The viral proteins, and/or T7 RNA polymerase, can also be provided from transformed mammalian cells, or by transfection of preformed mRNA or protein.

Alternatively, synthesis of antigenome or genome together with the above-mentioned viral proteins can be done in vitro (cell-free) in a combined transcription-translation reaction, followed by transfection into cells. Or, antigenome or genome RNA can be synthesized in vitro and transfected into cells expressing RSV proteins.

Having the infectious clone of the invention permits the alteration of the RSV genome (or antigenome) by introducing defined mutations. By "infectious clone" is meant cDNA or its product, synthetic or otherwise, which can be transcribed into genomic or antigenomic RNA capable of serving as template to produce the genome of infectious virus or subviral particle. Defined mutations can be introduced by conventional techniques (e.g., site-directed mutagenesis) into a cDNA copy of the genome or antigenome. The use of antigenome cDNA subfragments to assemble a complete antigenome cDNA as described herein has the advantage that each region can be manipulated separately (smaller cDNAs are easier to manipulate than large ones) and then readily assembled into a complete cDNA. Thus, the complete antigenome or genome cDNA, or any subfragment thereof, can be used as template for oligonucleotide-directed mutagenesis. This can be through the intermediate of a single-stranded phagemid form, such as using the Muta-gen kit of Bio-Rad, or a method using the doublestranded plasmid directly as template such as the Chameleon mutagenesis kit of Stratagene, or by the polymerase chain reaction employing either an oligonucleotide primer or template which contains the mutation(s) of interest. A mutated subfragment can then be assembled into the complete antigenome or genome cDNA. A variety of other mutagenesis techniques are known and available for use in producing the mutations of interest in the RSV antigenome or genome cDNA. Mutations can vary from single nucleotide changes to replacement of large cDNA pieces containing one or more genes or genome regions.

Thus, in one illustrative embodiment mutations are introduced by using the Muta-gene phagemid in vitro mutagenesis kit available from Bio-Rad Laboratories, Richmond, CA. In brief, cDNA encoding an RSV gnome or antigenome is cloned into the plasmid pTZ18U, and used to transform DH5α F' cells (Life Technologies Inc. , Gaithersburg, MD). Phagemid preparations are prepared as recommended by the manufacturer. Oligonucleotides are designed for mutagenesis by introduction of an altered nucleotide at the desired position of the genome or antigenome. The plasmid containing the genetically altered genome or antigenome is then amplified.

The ability to introduce defined mutations into infectious RSV has many applications, including the analyses of RSV molecular biology and pathogenesis. For example, the functions of the RSV proteins, including the NS1, NS2, SH, M2 (ORF1) and M2 (ORF2) proteins, can be investigated by introducing mutations which ablate or reduce their level of expression, or which yield mutant protein.

As another example, the sequence at the cleavage site of the F protein, or the putative attachment domain of the G protein can be modified to evaluate effects on growth in tissue culture and infection and pathogenesis in experimental animas.

The roles of various genome RNA structural features, such as promoters, intergenic regions, gene overlap, and transcription signals, can be evaluated using the methods and compositions of the present invention. Evaluation of trans-acting proteins and cis-acting RNA sequences using the complete antigenome cDNA can be conducted in parallel using RSV minigenomes (e.g., Grosfeld et al., J. Virol. 69: 5677-5686 (1995)), whose helper-dependent status is useful in the characterization of those mutants which are too inhibitory to be recovered in replication-independent infectious virus.

A number of attenuated RSV strains as candidate vaccines for intranasal administration have been developed using multiple rounds of chemical mutagenesis to introduce multiple mutations into a virus which had already been attenuated during cold-passage (e.g., Connors et al., Virology 208: 478-484 (1995); Crowe et al., Vaccine 12: 691-699 (1994); and Crowe et al., Vaccine 12: 783-790 (1994)). Evaluation in rodents, chimpanzees, adults and infants indicate that certain of these candidate vaccine strains are relatively stable genetically, are highly immunogenic, and may be satisfactorily attenuated. Nucleotide sequence analysis of some of these attenuated viruses indicates that each level of increased attenuation is associated with two or more new nucleotide and amino acid substitutions (Connors et al., supra). The present invention provides the ability to distinguish between silent incidental mutations versus those responsible for phenotype differences by introducing the mutations, separately and in various combinations, into the genome or antigenome of infectious wild-type RSV. This process identifies mutations responsible for phenotypes such as attenuation, temperature sensitivity, cold-adaptation, small plaque size, host range restriction, etc. Mutations from this menu can then be introduced in various combinations to calibrate a vaccine virus to an appropriate level of attenuation, etc., as desired. Moreover, the present invention provides the ability to combine mutations from different strains of virus into one strain.

The present invention also provides new methods of attenuation. For example, individual internal genes of human RSV can be replaced with their bovine, murine or other RSV counterpart. This may include part or all of one or more of the NS1, NS2, N, P, M, SH, M2(ORF1), M2(ORF2) and L genes, or non-immunogenic parts of the G and F genes. Reciprocally, means are provided to generate a live attenuated bovine RSV by inserting human attenuating genes into a bovine RSV genome or antigenome background. Human RSV bearing bovine RSV glycoproteins provides a host range restriction favorable for human vaccine preparations. Bovine RSV sequences which can be used in the present invention are described in, e.g., Pastey et al., J. Gen. Viol. 76:193-197 (1993); Pastey et al., Virus Res. 29:195-202. (1993); Zamora et al., J. Gen. Virol. 73 :737-741 (1992); Mallipeddi et al., J. Gen. Virol. 74:2001-2004 (1993); Mallipeddi et al., J. Gen. Virol. 73 :2441-2444 (1992); and Zamora et al., Virus Res. 24:115-121 (1992).

The invention also provides the ability to analyze other types of attenuating mutation and to incorporate the into infectious RSV for vaccine or other uses. For example, a tissue culture-adapted nonpathogenic strain of pneumonia virus of mice (the murine counterpart of RSV) lacks a cytoplasmic trail of the G protein (Randhawa et al., Virology 207: 240-245 (1995)). By analogy, the cytoplasmic and transmembrane domains of each of the RSV glycoproteins, F, G and SH, can be deleted or modified to achieve attenuation.

Other mutations for use in infectious RSV of the present invention include mutations in cis-acting signals identified during mutational analysis of RSV minigenome. For example, insertional and deletional analysis of the leader and trailer and flaking sequences identified viral promoters and transcription signals and provided a series of mutations associated with varying degrees of reduction of RNA replication or transcription. Saturation mutagenesis (whereby each position in turn is modified to each of the nucleotide alternatives) of these cis-acting signals also has identified many mutations which reduced (or in one case increased) RNA replication or transcription. Any of these mutations can be inserted into the complete antigenome or genome as described herein. Other mutations involved replacement of the 3' end of genome with its counterpart from antigenome, which is associated with changes in RNA replication and transcription. In addition, the intergenic regions (Collins et al., Proc. Natl. Acad. Sci. USA 83:4594-4598 (1986)) can be shortened or lengthened or changed in sequence content, and the naturally-occurring gene overlap (Collins et al., Proc. Natl. Acad. Sci. USA 84:5134-5138. (1987)) can be removed or changed to a different intergenic region by the methods described herein.

In another embodiment, RSV useful in a vaccine formulation can be conveniently modified to accommodate antigenic drift in circulating virus. Typically the modification will be in the G and/or F proteins. The entire G or F gene, or the segment(s) encoding particular immunogenic regions thereof, is incorporated into the RSV genome or antigenome cDNA by replacement of the corresponding region in the infectious clone or by adding one or more copies of the gene such that several antigenic forms are represented. Progeny virus produced from the modified RSV cDNA are then used in vaccination protocols against the emerging strains. Further, inclusion of the G protein gene of RSV subgroup B would broaden the response to cover a wider spectrum of the relatively diverse subgroup A and B strains present in the human population.

An infectious RSV clone of the invention can also be engineered to enhance its immunogenicity and induce a level of protection greater than that provided by natural infection, or vice versa, to identify and ablate epitopes associated with undesirable immunopathologic reactions. Enhanced immunogenicity of the vaccines produced by the present invention addresses one of the greatest obstacles to controlling RSV, namely the incomplete nature of immunity induced by natural infection. An additional gene may be inserted into or proximate to the RSV genome or antigenome which is under the control of an independent set of transcription signals. Genes of interest include those encoding cytokines (e.g., IL-2 through IL-15, especially IL-3, IL-6 and IL-7, etc.), gamma-interferon, and proteins rich in T helper cell epitopes. The additional protein can be expressed either as a separate protein or as a chimera engineered from a second copy of one of the RSV proteins, such as SH. This provides the ability to modify and improve the immune response against RSV both quantitatively and qualitatively.

For vaccine use, virus produced according to the present invention can be used directly in vaccine formulations, or lyophilized, as desired, using lyophilization protocols well known to the artisan. Lyophilized virus will typically be maintained at about 4°C. When ready for use the lyophilized virus is reconstituted in a stabilizing solution, e.g., saline or comprising SPG, Mg⁺⁺ and HEPES, with or without adjuvant, as further described below.

Thus RSV vaccines of the invention contain as an active ingredient an immunogenetically effective amount of RSV produced as described herein. The modified virus may be introduced into a host with a physiologically acceptable carrier and/or adjuvant. Useful carriers are well known in the art, and include, e.g., water, buffered water, 0.4% saline, 0.3% glycine, hyaluronic acid and the like. The resulting aqueous solutions may be packaged for use as is, or lyophilized, the lyophilized preparation being combined with a sterile solution prior to administration, as mentioned above. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, and the like. Acceptable adjuvants include incomplete Freund's adjuvant, aluminum phosphate, aluminum hydroxide, or alum, which are materials well known in the art.

Upon immunization with a RSV composition as described herein, via aerosol, droplet, oral, topical or other route, the immune system of the host responds to the vaccine by producing antibodies specific for RSV virus proteins, e.g., F and G glycoproteins. As a result of the vaccination the host becomes at least partially or completely immune to RSV infection, or resistant to developing moderate or severe RSV infection, particularly of the lower respiratory tract.

The host to which the vaccine are administered can be any mammal which is susceptible to infection by RSV or a closely related virus and which host is capable of generating a protective immune response to the antigens of the vaccinizing strain. Thus, suitable hosts include humans, non-human primates, bovine, equine, swine, ovine, caprine, lagamorph, rodents, etc. Accordingly, the invention provides methods for creating vaccines for a variety of human and veterinary uses.

The vaccine compositions containing the RSV of the invention are administered to a host susceptible to or otherwise at risk of RSV infection to enhance the host's own immune response capabilities. Such an amount is defined to be a "immunogenically effective dose." In this use, the precise amounts again depend on the host's state of health and weight, the mode of administration, the nature of the formulation, etc., but generally range from about 10³ to about 10⁶ plaque forming units (PFU) or more of virus per host, more commonly from about 10⁴ to 10⁵ PFU virus per host. In any event, the vaccine formulations should provide a quantity of modified RSV of the invention sufficient to effectively protect the host patient against serious or life-threatening RSV infection.

The RSV produced in accordance with the present invention can be combined with viruses of the other subgroup or strains to achieve protection against multiple RSV subgroups or strains, or protective epitopes of these strains can be engineered into one virus as described herein. Typically the different viruses will be in admixture and administered simultaneously, but may also be administered separately. For example, as the F glycoproteins of the two RSV subgroups differ by only about 11% in amino acid sequence, this similarity is the basis for a cross-protective immune response as observed in animals immunized with RSV or F antigen and challenged with a heterologous strain. Thus, immunization with one strain may protect against different strains of the same or different subgroup.

In some instances it may be desirable to combine the RSV vaccines of the invention with vaccines which induce protective responses to other agents, particularly other childhood viruses. For example, the RSV vaccine of the present invention can be administered simultaneously with parainfluenza virus vaccine, such as described in Clements et al., J. Clin. Microbiol. 29:1175-1182 (1991). In another aspect of the invention the RSV can be employed as a vector for protective antigens of other respiratory tract pathogens, such as parainfluenza, by incorporating the sequences encoding those protective antigens into the RSV genome or antigenome which is used to produce infectious RSV as described herein.

Single or multiple administrations of the vaccine compositions of the invention can be carried out. In neonates and infants, multiple administration may be required to elicit sufficient levels of immunity. Administration should begin within the first month of life, and at intervals throughout childhood, such as at two months, six months, one year and two years, as necessary to maintain sufficient levels of protection against native (wild-type) RSV infection. Similarly, adults who are particularly susceptible to repeated or serious. RSV infection, such as, for example, health care workers, day carer workers, family members of young children, the elderly, individuals with compromised cardiopulmonary function, may require multiple immunizations to establish and/or maintain protective immune responses. Levels of induced immunity can be monitored by measuring amounts of neutralizing secretory and serum antibodies and dosages adjusted or vaccinations repeated as necessary to maintain desired levels of protection. Further, different vaccine viruses may be advantageous for different recipient groups. For example, an engineered RSV strain expressing an additional protein rich in T cell epitopes may be particularly advantageous for adults rather than for infants.

In yet another aspect of the invention the RSV is employed as a vector for transient gene therapy of the respiratory tract. According to this embodiment the recombinant RSV genome or antigenome incorporates a sequence which is capable of encoding a gene product of interest. The gene product of interest is under control of the same or a different promoter from that which controls RSV expression. The infectious RSV produced by coexpressing the recombinant RSV genome or antigenome with the N, P, L and M2(ORF1) proteins and containing a sequence encoding the gene product of interest is administered to a patient. Administration is typically by aerosol, nebulizer, or other topical application to the respiratory tract of the patient being treated. Recombinant RSV is administered in an amount sufficient to result in the expression of therapeutic or prophylactic levels of the desired gene product. Examples of representative gene products which are administered in this method include those which encode, for example, those particularly suitable for transient expression, e.g., interleukin-2, interleukin-4, gamma-interferon, GM-CSF, G-CSF, erythropoietin, and other cytokines, glucocerebrosidase, phenylalanine hydroxylase, cystic fibrosis transmembrane conductance regulator (CFTR), hypoxanthine-guanine phosphoribosyl transferase, cytotoxins, tumor suppressor genes, antisense RNAs, and vaccine antigens.

The following examples are provided by way of illustration, not limitation.

### EXAMPLE I

### Construction of cDNA Encoding RSV Antigenome

A cDNA clone encoding the antigenome of RSV strain A2 was constructed, as shown in Fig. 1A. The cDNA was synthesized in segments by reverse transcription (RT) and polymerase chain reaction (PCR) using synthetic oligonucleotides as primers and intracellular RSV mRNA or genome RNA isolated from purified virions as template. The final cDNA was flanked on the leader end by the promoter for T7 RNA polymerase, which included three transcribed G residues for optimal activity; transcription would result in the donation of these three nonviral G's to the 5' end of the antigenome. To generate a nearly-correct 3' end, the cDNA trailer end was constructed to be adjacent to a previously-described hammerhead ribozyme, which upon cleavage would donate a single 3'-phosphorylated U residue to the 3' end of the encoded RNA (Grosfeld et al., J. Virol. 69:5677-5686). The ribozyme sequence was followed by a tandem pair of terminators of T7 RNA polymerase. (The addition of three 5' G residues and one 3' U residue to a cDNA-encoded RSV minigenome containing the chloramphenicol acetyl transferase (CAT) reporter gene had no effect on the expression of CAT when complemented by RSV.)

Fig. 1A shows the structures of the cDNA and the encoded antigenome RNA (not to scale). The diagram of the antigenome (at top) includes the following features: the 5' -terminal nonviral G triplet contributed by the T7 promoter, the.four sequence markers at positions 1099 (which adds one nt to the length), 1139, 5611, and 7559, the ribozyme and tandem T7 terminators, and the single nonviral 3'-phosphorylated U residue contributed to the 3' end by ribozyme cleavage. (the site of cleavage is indicated with an arrow) (13).

Cloned cDNA segments (Fig. 1A, middle) representing in aggregate the complete antigenome were constructed by RT-PCR of RSV mRNA or genome RNA. cDNAs containing the lefthand end of the antigenome, spanning from the T7 promoter and leader region complement to the SH gene, were assembled in a version of pBR322 (Fig. 1A, bottom) in which the naturally-occurring BamHI site had been ablated by mutagenesis and the PstI-EcoRI fragment replaced with a synthetic polylinker containing unique restriction sites (including BstBI, BstXI, PacI, BamHI, MluI) designed to facilitate assembly. The box in Fig. 1A shows the removal of the BamHI site. The naturally occurring BamHI-SalI fragment (the BamHI site is shown in the top line in positive sense, underlined) was replaced with a PCR-generated BglII-SalI fragment (the BglII site is shown in the bottom line, underlined; its 4-nt sticky end [italics] is compatible with that of BamHI). This resulted in a single nt change (middle line, underlined) which was silent at the amino acid level. These modifications to the vector facilitated construction of the cDNA by rendering unique a BamHI site in the antigenome cDNA.

The G, F and M2 genes were assembled in a separate plasmid, as were the L, trailer and flanking ribozyme and tandem T7 transcription terminators. The G-to-M2 piece was then inserted into the PacI-BamHI window of the leader-to-SH plasmid. This in turn was the recipient for the L-trailer-ribozyme-terminator piece inserted into the BamHI to MluI, yielding the complete antigenome.

Four restriction site markers (Fig. 1B) were introduced into the antigenome cDNA by incorporating the changes into oligonucleotide primers used in RT-PCR. This was done to facilitate assembly, provide a means to identify recombinant virus, and illustrate the ability to introduce changes into infectious RSV. Three sites were in intergenic regions and the fourth in a nontranslated gene region, and they involved a total of five nt substitutions and a single nt insertion. This increased the length of the encoded antigenome by one nt from that of wild type to a total of 15,223 nt (SEQ ID NO:1, which depicts the 5' to 3' positive-sense sequence whereas the genome itself is negative-sense).

The sequence markers were inserted into the cDNA-encoded antigenome RNA as shown in Fig. 1B. Sequences are positive sense and numbered relative to the first nt of the leader region complement as 1; identities between strains A2 and 18537 (Johnson and Collins, J. Gen Virol. 69:2901-2906 (1988)), representing subgroups A and B, respectively, are indicated with dots; sequences representing restriction sites in the cDNA are underlined; gene-start (GS) and gene-end (GE) transcription signals are boxed; the initiation codon of the N translational open reading frame at position 1141 is italicized, and the sequence markers are shown underneath each sequence. In the top sequence, a single C residue was inserted at position 1099 to create an AflII site in the NSII-N intergenic region, and the AG at positions 1139 and 1140 immediately upstream of the N translational open reading frame were replaced with CC to create a new NcoI site. In the middle sequence, substitution of G and U at positions 5612 and 5616, respectively, created a new StuI site in the G-F intergenic region. And, in the bottom sequence of Fig. 1B, a C replacement at position 7561 created a new SphI site in the F-M2 intergenic region.

All cDNAs were sequenced in their entirety, in most instances from several independent cDNAs, prior to assembly. The plasmids encoding individual RSV proteins are described in Grosfeld et al., J. Virol. 69:5677-5686 (1995) and Collins et al., Proc. Natl. Acad. Sci. USA (1995).

### EXAMPLE II

### Transfection and Recovery of Recombinant RSV

The strategy for producing infectious RSV from cDNA-expressed antigenome involved its coexpression with those RSV proteins which are sufficient to (i) produce an antigenome nucleocapsid capable of RNA replication, and (ii) renders the progeny genome nucleocapsid competent for both RNA replication and transcription. Transcription by the genome nucleocapsid provides all of the other RSV proteins and initiates a productive infection.

Plasmid-borne cDNA encoding the antigenome was transfected, together with plasmids encoding proteins N, P, L and M2(ORF1), into HEp-2 cells which had been infected with a recently-described vaccinia virus MVA strain recombinant which expresses the T7 RNA polymerase (Wyatt et al., Virol. 210:202-205 (1995)). The MVA strain is a host range mutant which grows permissively in avian cells whereas in mammalian cells there is a block at a late stage in virion maturation that greatly reduces the production of infectious virus. In HEp-2 cells, the MVA recombinant was similar to the more commonly-used WR-based recombinant (Fuerst et al., Proc. Natl. Acad. Sci. USA 83: 8122-8126 (1986)) with regard to the level of expression of T7 polymerase and cytopathogenicity, but the level of progeny produced was sufficiently low that supernatants could be passaged to fresh cells with minimal cytopathogenicity. This should facilitate the recovery of any recombinant RSV which might be produced in transfected, vaccinia virus-infected cells.

Transfection and recovery of recombinant RSV was performed as follows. Monolayer cultures of HEp-2 cells received, per single well of a six-well dish, one ml of infection-transfection medium prepared by mixing five plasmids in a final volume of 0.1 ml Opti-MEM (Life Technologies) medium, namely 0.4 µg each of antigenome, N and P plasmids, and 0.1 µg each of L and M2(ORF1) plasmids. This was combined with 0.1 ml of Opti-MEM containing 12 µl LipofectACE (Life Technologies). After 15 min incubation at room temperature, this was combined with 0.8 ml of OptiMEM containing 2% heat-inactivated fetal calf serum and 1.5 X 10⁶ pfu of strain MVA vaccinia virus recombinant encoding T7 RNA polymerase (Wyatt et al., supra). This was added to the cells and replaced one day later by Opti-MEM containing 2% serum. Cultures were incubated at 32°C and harvested on day three. Incubation at 32°C was used because it was found that the MVA virus is slightly temperature sensitive and is much more efficient at this lower temperature.

Three days post-transfection clarified culture supernatants were passaged onto fresh HEp-2 cells and overlaid with methyl cellulose (for subsequent antibody staining) or agarose (for plaque isolation). After incubation for five days under methyl cellulose, the cells were fixed and stained by an indirect horseradish peroxidase method using a mixture of three murine monoclonal antibodies to the RSV F protein followed by an anti-mouse antibody linked to horseradish peroxidase, following the general procedure of Murphy et al., Vaccine 8: 497-502 (1990).

Numerous RSV-like plaques were detected against a background of cytopathogenicity that presumably was due to a low level of MVA-T7 recombinant virus. The plaques contained an abundant amount of the RSV F protein, as indicated by brown-black coloration, and displayed cytopathic effects characteristic of RSV, notably syncytium formation.

The RSV-like plaques were picked from plates which had been prepared in parallel but incubated under agarose and stained with neutral red. They were propagated and compared to a laboratory strain of RSV strain A2 by plaque assay and antibody staining. The plaques derived from the transfected cultures closely resembled those of the laboratory strain. One difference was that the plaques derived from the transfected cultures appeared to be slightly smaller than those from the laboratory strain, with centers which were less well cleared. The recombinant virus may differ phenotypically from this particular wild type isolate, possibly being slightly more restricted in cell-to-cell spread and exhibiting a reduced rate of cell killing. With regard to the propagation of released virus, the yields of the recombinant versus laboratory virus in HEp-2 cells were essentially identical at 32° or 37°C. In preliminary studies, the recombinant and laboratory viruses were indistinguishable with regard to the accumulation of intracellular RSV mRNAs and proteins.

Plaque-purified, thrice-passaged recombinant RSV was analyzed in parallel with laboratory virus by RT-PCR using three primer pairs flanking the four inserted markers. Three independent plaque-purified recombinant RSV isolates were propagated in parallel with an uninfected control culture. Clarified medium supernatants were treated with polyethylene glycol and high salt (Zoller and Smith, DNA 3:479-488 (1984)) to precipitate virus and RNA was extracted from the pellets with TrizolTM (Life Technologies). These RNAs, in parallel with additional controls of no added RNA or 0.1 µg of RNA from a laboratory isolate of strain A2, were treated with DNAse, repurified, annealed each with 50 ng of random hexamers and incubated under standard RT conditions (40 µl reactions) with or without reverse transcriptase (Connors et al., Virol. 208: 478-484 (1995)). Aliquots of each reaction were subjected to PCR (35 cycles of 94°C for 45s, 37°C for 30s, 72°C for 1 min) using three different pairs of synthetic deoxyoligonucleotide primers. Primer pair (A): positive-sense, positions 925-942 and negative-sense, positions 1421-1440, yielding a predicted product of 516 bp (517 bp in the case of the recombinant viruses) that included the AflII and NcoI sites inserted at, respectively, the junction of the NS2 and N genes and in the N gene. Primer pair (B): positive-sense, positions 5412-5429 and negative-sense, 5930-5949, yielding a predicted product of 538 bp spanning the StuI site inserted at the junction between the G and F genes. Primer pair (C): positive-sense, 7280-7297 and negative-sense, 7690-7707, yielding a 428 bp fragment spanning the SphI site inserted at the junction between the F and M2 genes. PCR products were analyzed by electrophoresis on neutral gels containing 1% agarose and 2% low-melting agarose in parallel with HaeIII-digested X174 DNA molecular length markers and visualized by staining with ethidium bromide. PCR products of the expected sizes were produced. The production of each was dependent on the RT step, indicating that each was derived from RNA rather than contaminating cDNA.

PCR products were analyzed by digestion with restriction enzymes. Digestion of products of primer pair A with AflII or NcoI yielded fragments corresponding to the predicted 177 and 340 bp (AflII) or 217 and 300 bp (NcoI). Digestion of products of primer pair B with StuI yielded fragments comparable to the predicted 201 and 337 bp. Digestion of products from reactions with primer pair C with SphI yielded products corresponding to the predicted 147 and 281 bp. The digests were analyzed by gel electrophoresis as above. The presence of residual undigested PCR product with AflII was due to incomplete digestion, as was confirmed by redigestion. Thus, the restriction enzyme digestion showed that the PCR products representing recombinant virus contained the expected restriction site markers while those representing the laboratory strain did not. Nucleotide sequence analysis of cloned PCR product confirmed the sequences spanning the restriction site markers.

As shown in Table 1, the efficiency of RSV production when complemented by N, P, L and M2 (ORF1) was relatively high, ranging in three experiments from an average of 9.9 to 94.8 plaques per 0.4 µg of input antigenome cDNA and 1.5 X 10⁶ cells. Since these plaques were derived from passage, the number of infected cells present in each well of the original transfection was not known. Nearly every transfected well (54 of 56 in Table 1) produced virus. Since the yield of released RSV per infected cell typically is very low (∼10 pfu) even under ideal conditions, and since many wells yielded many times this amount (up to 169 plaques in Table 1), it is likely that several RSV producing cells were present in many of the wells of transfected cells.

RSV was not recovered if any of the plasmids were omitted (e.g., as shown in Table 1). The requirement for M2(ORF1) also could be satisfied with the complete gene, M2(ORF1+2), provided the level of its input cDNA was low (0.016 µg per 1.5 X 10⁶ cells [Table 1]). At higher levels, the production of virus was greatly reduced, suggesting that an inhibition of minigenome RNA synthesis associated with M2(ORF2) also operates on the complete genome during productive infection.

These results showed that the production of infectious RSV was highly dependent on expression of the M2(ORF1) protein in addition to N, P and L. Furthermore, it showed that the optimal method of expression of M2(ORF1) was from an engineered cDNA in which ORF2 had been deleted, although the complete cDNA containing both ORFs also supported the production of RSV.

Thus, as part of the present invention, transcription by RSV differed from previously-described nonsegmented negative strand RNA viruses in requiring a fourth protein designated here as M2(ORF1), and previously called 22K or M2 (Collins et al., J. Virol. 54:65-71 (1985)). The M2(ORF1) protein was found to be an RNA polymerase elongation factor essential for processive, sequential transcription. This requirement provides the capability, as part of this invention, for introducing specific, predetermined changes into infectious RSV.

**Table 1. Production of infectious RSV was dependent on expression of M2 ORF 1.**

| Complementing plasmids (µg cDNA per 1.5X10⁶ cells and antigenome cDNA) 0.4 µg | Production of infectious RSV #plaques X # wells* | | | |
|---|---|---|---|---|
| | expt. 1 | | expt. 2 | expt. 3 |
| N(0.4) | | | | |
| P(0.4) | 0 X 24 | | 0 X 12 | 0 X 12 |
| L(0.1) | | | | |
| | | | | |
| N(0.4) | 0 X 19^{§} | | 0 X 4 | 9 X 1 |
| P(0.4) | 1 X 2 | | 3 X 1 | 10 X 1 |
| L(0.1) | 2 X 2 | | 5 X 1 | 14 X 2 |
| M2 [ORF1+2] (0.016) | 3 X 1 | | 6 X 1 | 22 X 1 |
| | | | 9 X 1 | 28 X 1 |
| | av. 0.38 | | 10 X 1 | 32 X 1 |
| | | | 13 X 1 | 49 X 1 |
| | | | 34 X 1 | 70 X 2 |
| | | | 51 X 1 | 166 X 1 |
| | | | | 169 X 1 |
| | | | av. 10.9 | |
| | | | | av. 48.6 |
| | | | | |
| N(0.4) | 0 X 1 | 11 X | 1 0 X 1 | 55 X 1 |
| P(0.4) | 1 X 1 | 12 X 1 | 2 X 1 | 59 X 1 |
| L(0.1) | 2 X 2 | 13 X 1 | 4 X 1 | 65 X 1 |
| M2 [ORF1] (0.1) | 3 X 2 | 21 X 1 | 5 X 1 | 71 X 1 |
| | 4 X 1 | 24 X 1 | 8 X 2 | 72 X 1 |
| | 5 X 2 | 26 X 1 | 10 X 3 | 87 X 1 |
| | 6 X 4 | 30 X 2 | 19 X 1 | 97 X 1 |
| | 7 X 2 | 33 X 2 | 20 X 1 | 100 X 1 |
| | 9 X 1 | 42 X 1 | 23 X 1 | 109 X 1 |
| | 10 X 2 | 73 X 1 | av. 9.9 | 128 X 1 147 X 1 |
| | | av. 13.7 | | 148 X 1 |
| | | | | av. 94.8 |

| | | | | |
|---|---|---|---|---|
| Supernatants from transfected cultures (10⁶ cells per well) were passaged onto fresh HEp-2 cells, overlaid with methyl cellulose, and stained with F-specific monoclonal antibodies. ^{§} Read as follows: 19 wells had 0 plaques, 2 wells had 1 plaque each, 2 wells had 2 plaques each, and 1 well had 3 plaques. | | | | |

### EXAMPLE III

### Constructing Infectious RSV With Predetermined Mutations To Confer a Desired Phenotype

This Example illustrates the introduction of specific predetermined mutations into infectious recombinant RSV using the methods described hereinabove. For ease of manipulation, the antigenome cDNA was cloned as two separate pieces in separate plasmids: one piece (the left end) containing the T7 promoter together with nucleotide 1 through to the BamHI site at nucleotide 8501 (cDNA D50), and the other (the right end) containing the BamHI site through to nucleotide 15223, together with the ribozyme and T7 transcription terminators (cDNA D39). D39 was further separated into two pieces and each placed in a separate phagemid plasmids: one piece (left hand half, cDNA LI) from the BamHI site to the PmlI- site at nucleotide 12255, and the other (right hand half, cDNA L2) from the PmlI site to the end of the T7 terminator. The sequence positions assigned to restriction site locations are intended as a descriptive guide and do not alone precisely define all of the nucleotides involved.

Following a general procedure of Kunkel et al. Meth. Enzymol. 54:367-382 (1987)), the plasmids were propagated in a dut ung strain of E. coli, strain CJ236, and single stranded DNA was prepared by infection with a helper phage, M13KO7. Phosphorylated synthetic oligonucleotides each containing one or more nucleotides changes of interest were prepared, annealed to the single stranded template singly or more than one at a time, and used to direct DNA synthesis by T4 DNA polymerase. The products were ligated and transformed into a non-dut ung strain of E. coli, DH5α or DH10B. Colonies containing mutant plasmids were identified by restriction enzyme digestion or by sequence analysis. Other methods of mutagenesis can readily be used.

L1 and L2 described above were modified to contain several combinations of recognition sites for several different restriction enzymes which do not appear or are infrequent in the antigenome plasmid; these sites were introduced using nucleotide substitutions which did not change the amino acid sequence of the encoded L protein. In addition, L1 was modified to contain a mutation believed to confer a ts phenotype. Two versions of L1 were made. In one version, L1 was modified in a single cycle of mutagenesis to contain new Bsu36I (nucleotide 9399) and SnaBI (11848) sites, and a mutation termed 530, yielding cDNA 530Llsites. The 530 mutation had been identified by sequence analysis of the biologically-derived virus cpts530-RSV and involves a single nucleotide change at position 10060 which results in an amino acid change (Phe to Leu) at amino acid 521 in the L protein. In a second version, L1 was modified in a single cycle to contain the Bsu36I and SnaBI sites, resulting in cDNA L1 sites. L2 was modified in one cycle of mutagenesis to contain the new sites PmeI (13342), RsrII (14083) and SnaBI (14477). In a second cycle, the site BstEII (14318) was added and a naturally-occurring recognition site for SnaBI (6956) was removed. This yielded L2 sites.

Three complete antigenome cDNAs were made by introducing selected L1 and L2 mutant cDNAs or fragments thereof into D39 and combining this with D50. Antigenome cDNA "D53sites" contains L1 sites and L2 sites. cDNA "530D53" contains the BamHI-SpeI (10149) fragment of 530L1sites (which contains the Bsu36I and 530 mutations). cDNA "530D53sites" contained 530L1 sites and L2 sites (Table II). Recombinant virus was recovered from each of the three complete mutant antigenome cDNAs using the methodology of this invention and were passaged at least twice and analyzed directly or following plaque purification and amplification. The presence of mutations was confirmed by RT-PCR of viral RNA followed by analysis by restriction enzyme digestion or nucleotide sequencing, or both.

The engineered viruses were evaluated for their ability to form plaques in HEp-2 cells at 32°C, 39°C and 40°C in parallel with two nonrecombinant biologically-derived viruses, HEK, a wild type strain A2 virus, and cpts530 RSV, the virus from which the 530 mutation was identified by sequence analysis (Table II). This comparison showed that all three engineered viruses formed plaques at 32°C, and showed that the titers of the various virus preparations were within two log₁₀ units of each other, which is within the range of experimental variation typically seen among independent preparations of RSV. The recombinant viruses containing the 530 mutation were greatly impaired in ability to form plaques at 39°C or 40°C, comparable to cpts530 RSV. The presence of additional restriction sites in 530D53sites versus 530D53 had no discernable effect on the ts phenotype. D53sites virus, which contained silent restriction site markers but lacked the 530 mutation, retained the ability to form plaques at the higher temperatures, comparable to wild type. This not only provided positive identification that the 530 mutation is involved in the ts phenotype of cpts530 RSV, but also showed that point mutations can be introduced systematically into recombinant RSV according to the present invention. In these cases, the resulting phenotypes of the engineered viruses were fully consistent with the parental strain and provided direct confirmation and reconstitution of an attenuation mutation.

**Table II**

| Characterization of the ts phenotype of biologically-derived RSV versus RSV recovered from cDNA clones | | | |
|---|---|---|---|
| virus | Efficiency of plaque formation (log₁₀) at the indicated temperature | | |
| | 32°C | 39°C | 40°C |
| Biologically-derived viruses | | | |
| HEK^{a} | 8.7 | 8.6 | 8.5 |
| cpts530RSV^{b} | 6.8 | <0.7 | <0.7 |

| cDNA-derived viruses | | | |
|---|---|---|---|
| D53 sites^{c} | 6.9 | 6.9 | 6.7 |
| 530DS3^{d} | 7.9 | <0.7 | <0.7 |
| 530D53 sites^{e} | 7.6 | <0.7 | <0.7 |

| | | | |
|---|---|---|---|
| ^{a} = Wild type RSV A2. ^{b} = ts virus. ^{c} = Contains six new restriction sites and lacks one naturally-occurring site. ^{d} = Contains one new restriction site and the 530 mutation. ^{e} = Contains six new restriction sites and the 530 mutation and lacks one naturally-occurring site. | | | |

### EXAMPLE IV

### Recovery of Infectious Respiratory Syncytial Virus Expressing An Additional, Foreign Gene

The methods described above were used to construct recombinant RSV containing an additional gene, encoding chloramphenicol acetyl transferase (CAT). The CAT coding sequence was flanked by RSV-specific gene-start and gene-end motifs, the transcription signals for the viral RNA-dependent RNA polymerase. The RSV/CAT chimeric transcription cassette was inserted into the intergenic region between the G and F genes of the complete cDNA-encoded positive-sense RSV antigenome, and infectious CAT-expressing recombinant RSV was recovered. The CAT mRNA was efficiently expressed and the levels of the G and F mRNAs were comparable to those expressed by wild type recombinant RSV. The CAT-containing and wild type viruses were similar with regard to the levels of synthesis of the major viral proteins.

Plasmid D46 was used for construction of cDNA encoding RSV antigenomic RNA containing the CAT gene. (Plasmids D46 and D50, the latter mentioned in Example III, are different preparations of the same antigenome cDNA.) D46 which encodes the compete, 15,223-nucleotide RSV antigenome (one nucleotide longer than that of wild type RSV) and was used to produce recombinant infectious RSV described above. During its construction, the antigenome cDNA had been modified to contain four new restriction sites as markers. One of these, a *Stu*I site placed in the intergenic region between the G and F genes (positions 5611-5616 in the 3'-5' sequence of the wild type genome), was chosen as an insertion site for the foreign CAT gene. A copy of the CAT ORF flanked on the upstream end by the RSV GS signal and on the downstream end by the RS GE signal was derived from a previously-described RSV-CAT minigenome (Collins et al., Proc. Natl. Acad. Sci. USA 88:9663-9667 (1991) and Kuo et al., J. Virol. 70: 6892-6901 (1996)). The insertion of this RSV/CAT transcription cassette into the *Stu*I site, to yield the D46/1024CAT cDNA, increased the length of the encoded antigenome to a total of 15,984 nucleotides. And; whereas wild type RSV encodes ten major subgenomic mRNAs, the recombinant virus predicted from the D46/1024CAT antigenome would encode the CAT gene as an eleventh mRNA. The strategy of construction is shown in Fig. 2.

Producing infectious RSV from cDNA-encoded antigenomic RNA, as described above, involved coexpression in HEP-2 cells of five cDNAs separately encoding the antigenomic RNA or the N, P, L or M2(ORF1) protein, which are necessary and sufficient for viral RNA replication and transcription. cDNA expression was driven by T7 RNA polymerase supplied by a vaccinia-T7 recombinant virus based on the MVA strain. The MVA-T7 recombinant virus produced infectious progeny sufficient to cause extensive cytopathogenicity upon passage, and therefore, cytosine arabinoside, an inhibitor of vaccinia virus replication, was added 24 h following the transfection and maintained during the first six passages.

Two antigenome cDNAs were tested for the recovery of RSV: the D46 cDNA, and the D46/1024CAT cDNA. Each one yielded infectious recombinant RSV. Cells infected with the D46/ 1024CAT recombinant virus expressed abundant levels of CAT enzyme. For each virus, transfection supernatants were passaged to fresh cells, and a total of eight serial passages were performed at intervals of five to six days and a multiplicity of infection of less than 0.1 PFU per cell.

The CAT sequence in the D46/1024CAT genome was flanked by RSV GS and GE signals, and thus should be expressed as an additional, separate, polyadenylated mRNA. The presence of this predicted mRNA was tested by Northern blot hybridization of RNA from cells infected with D46/1024CAT virus or D46 virus at the eighth passage. Hybridization with a negative-sense CAT-specific riboprobe detected a major band which was of the appropriate size to be the predicted CAT mRNA, which would contain 735 nucleotides not including poly(A). This species was completely retained by oligo(dT) latex particles, showing that it was polyadenylated. In some cases, a minor larger CAT-specific species was detected which was of the appropriate size to be a G-CAT readthrough mRNA. The D46/1024CAT virus had been subjected to eight passages at low multiplicity of infection prior to the infection used for preparing the intracellular RNA. There was no evidence of shorter forms of the CAT mRNA, as might have arisen if the CAT gene was subject to deletion.

Replicate blots were hybridized with negative-sense riboprobe specific to the CAT, SH, G or F gene, the latter two genes flanking the inserted CAT gene. The blots showed that the expression of the subgenomic SH, G and F mRNAs was similar for the two viruses. Phosphoimagery was used to compare the amount of hybridized radioactivity in each of the three RSV mRNA bands for D46/1024CAT and D46. The ratio of radioactivity between D46/1024CAT and D46 was determined for each mRNA: SH, 0.77; G, 0.87; and F, 0.78. The deviation from unity probably indicates that slightly less RNA was loaded for D46/1024CAT versus D46, although it also is possible that the overall level of mRNA accumulation was slightly less for D46/1024CAT RSV. The demonstration that the three ratios were similar confirms that the level of expression of each of these mRNAs was approximately the same for D46/1024CAT versus D46. This, the insertion of the CAT gene between the G and F genes did not drastically affect the level of transcription of either gene.

To characterize viral protein synthesis, infected HEp-2 cells were labeled with [³⁵S]methionine, and cell lysates were analyzed by PAGE either directly or following immunoprecipitation under conditions where recovery of labeled antigen was essentially complete. Precipitation with a rabbit antiserum raised against purified RSV showed that the D46/1024CAT and D46 viruses both expressed similar amounts of the major viral proteins F₁, N, P, M, and M2. That a similar level of M2 protein was recovered for each virus was noteworthy because its gene is downstream of the inserted CAT gene. Accumulation of the F protein, which is encoded by the gene located immediately downstream of the insertion, also was examined by immunoprecipitation with a mixture of three anti-F monoclonal antibodies. A similar level of the F₁ subunit was recovered for each virus. Phosphorimagery analysis of the major viral proteins mentioned above was performed for several independent experiments and showed that some sample-to-sample variability, but overall the two viruses could not be distinguished on the basis of the level of recovered proteins. Precipitation with anti-CAT antibodies recovered a single species for the D46/1024CAT but not for the D46 virus. Analysis of the total labeled protein showed that the N, P and M proteins could be detected without immunoprecipitation (although detection of the latter was complicated by its comigration with a cellular species) and confirmed that the two viruses yielded similar patterns. The position corresponding to that of the CAT protein contained more radioactivity in the D46/1024CAT pattern compared to that of D46, as was confirmed by phosphorimagery of independent experiments. This suggested that the CAT protein could be detected among the total labeled proteins without precipitation, although this demonstration was complicated by the presence of a comigrating background band in the uninfected and D46-infected patterns.

RT-PCR was used to confirm the presence of the CAT gene in the predicted location of the genome of recombinant RSV. Total intracellular RNA was isolated from the cell pellet of passage eight of both D46/1024CAT and D46 RSV. Two primers were chosen that flank the site of insertion, the *Stu*I restriction endonuclease site at RSV positions 5611-5616: the upstream positive-sense primer corresponded to positions 5412-5429, and the downstream negative-sense one to positions 5730-5711. The positive-sense primer was used for the RT step, and both primers were included in the PCR.

RT-PCR of the D46 virus yielded a single product that corresponded to the predicted fragment of 318 nucleotides, representing the G/F gene junction without additional foreign sequence. Analysis of D46/1024CAT viral RNA yielded a single product whose electrophoretic mobility corresponded well with the predicted 1079 nucleotide fragment, representing the G/F gene junction containing the inserted CAT transcription cassette. The latter PCR yielded a single major band; the absence of detectable smaller products indicated that the population of recombinant genomes did not contain a large number of molecules with a deletion in this region. When PCR analysis was performed on D46/1024CAT virus RNA without the RT step, no band was seen, confirming that the analysis was specific to RNA. Thus, the RT-PCR analysis confirmed the presence of an insert of the predicted length in the predicted location in the genomic RNA of the D46/1024CAT recombinant virus.

Enzyme expression was used to measure the stability of the CAT gene. Cell pellets from all of the passages beginning with the third were tested for CAT expression. For the virus D46/1024CAT, all these assays displayed conversion of [¹⁴C] labeled chloramphenicol into acetylated forms. To investigate stability of expression, virus from 20 or 25 individual plaques from passage three or eight, respectively, was analyzed for CAT expression. All samples were positive, and the level of expression of CAT was similar for each of the 25 isolates from passage eight, as judged by assay of equivalent aliquots of cell lysate. This demonstrated that the activity of the CAT protein encoded by each isolate remained unimpaired by mutation.

To determine plaque morphology and size, beginning with the second passage, one-eighth of the medium supernatant (i.e., 0.5 ml) harvested from each passage stage was used to infect fresh HEp-2 cells in six-well plates that were incubated under methylcellulose overlay for five to seven days. The cells were then fixed and stained by incubation with monoclonal antibodies against RSV F protein followed by a second antibody linked to horseradish peroxidase. Earlier, it had been observed that recombinant RSV produced from cDNA D46 was indistinguishable from a naturally-occurring wild type RSV isolate with regard to efficiency of plaque formation over a range of temperatures in vitro, and the ability to replicate and cause disease when inoculated into the respiratory tract of previously uninfected chimpanzees. Thus, the D46 recombinant RSV was considered to be a virulent wild type strain. The plaques produced by the D46 and D46/1024CAT recombinant viruses were compared by antibody staining. Plaque morphology was very similar for the two viruses, although the average diameter of the CAT-containing recombinant plaques was 90 percent of that of the D46 virus, based on measurement of thirty randomly-selected plaques for each virus.

The efficiency of replication in tissue culture of the D46 and D46/1024CAT viruses was compared in a single step growth cycle. Triplicate monolayers of cells were infected with either virus, and samples were taken at 12 h intervals and quantitated by plaque assay. The results showed that the production of D46/1024CAT virus relative to D46 was delayed and achieved a maximum titer which was 20-fold lower.

These results show that it is possible to construct recombinant, helper-independent RSV expressing a foreign gene, in this instance the CAT gene. The recombinant RSV directed expression of the predicted polyadenylated subgenomic mRNA that encoded CAT protein, the protein being detected both by enzyme assay and by radioimmunoprecipitation. Other examples have produced RSV recombinants with the luciferase gene inserted at the same CAT site, or with the CAT or luciferase genes inserted between the SH and G genes. These viruses also exhibit reduced growth, whereas the numerous wild type recombinant viruses recovered exhibit undiminished growth. This indicates that the reduced growth indeed is associated with the inserted gene rather than being due to chance mutation elsewhere in the genome. The finding that insertion of a foreign gene into recombinant RSV reduced its level of replication and was stable during passage in vitro suggests that this provides yet another means for effecting attenuation for vaccine use. And, these results demonstrate that the methodology described herein is capable of recovering a virus that is restricted in growth.

These results also illustrate an advantage of the strategy of gene expression of the nonsegmented negative strand viruses, namely that the foreign coding sequences can be introduced as a separate transcription cassette that is expressed as a separate mRNA. The results also show that RSV can tolerate an increase of genome length of 762 nucleotides in the case of the CAT gene to a total of 15,984 nucleotides (1.05 times that of wild type RSV). The luciferase gene that was successfully recovered is almost three times longer.

The viral RNA-dependent RNA polymerases are known to have an error-prone nature due to the absence of proofreading and repair mechanisms. In RNA virus genomes, the frequency of mutation is estimated to be as high as 10⁻⁴ - 10⁻⁵ per site on average (Holland et al., Curr. Top. Microbiol. Immunol. 176:1-20 (1992) and references therein). In the case of the recombinant D46/1024CAT RSV produced here, correct expression of the foreign gene would be irrelevant for virus replication and would be free to accumulate mutations. The passages described here involved a multiplicity of infection less than 0.1 PFU per cell, and the duration of each passage level indicated that multiple rounds of infection were involved. While yields of infectious virus from RSV-infected tissue culture cells typically are low, intracellular macromolecular synthesis is robust, and the poor yields of infectious virus seems to represent an inefficient step in packaging rather than low levels of RNA replication. Thus, the maintenance of CAT through eight serial passages involved many rounds of RNA replication. It was surprising that the nonessential CAT gene remained intact and capable of encoding fully functional protein in each of the 25 isolates tested at the eighth passage. Also, RT-PCR analysis of RNA isolated from passage eight did not detect deletions within the CAT gene.

Because most of the antigenic difference between the two RSV antigenic subgroups resides in the G glycoprotein, recombinant RSV can be constructed to express the G protein of the heterologous subgroup as an additional gene to yield a divalent vaccine. Envelope protein genes of some other respiratory viruses, such as human parainfluenza 3 virus, also can be inserted for expression by recombinant RSV. Other uses include coexpression of immune modulators such as interleukin 6 to enhance the immunogenicity of infectious RSV. Other uses, such as employing modified RSV as described herein as a vector for gene therapy, are also provided.

Although the foregoing invention has been described in some detail by way of illustration and example for purposes of clarity of understanding, it will be obvious that certain changes and modifications may be practiced within the scope of the appended claims.

## Claims

1. A method for producing an infectious RSV particle from one or more polynucleotide molecules encoding said RSV, comprising:
coexpressing in a cell or cell-free lysate an expression vector which comprises a polynucleotide molecule encoding a RSV genome or antigenome, wherein the recombinant RSV genome or antigenome is recombinantly modified by the introduction of a mutation by insertion, rearrangement, deletion or substitution of one or more nucleotides, said mutation producing a phenotype of attenuation, temperature sensitivity, tissue culture adaption, host range restriction or an enhanced immunogenicity of the RSV particle compared to wild-type RSV virus, and an expression vector which comprises one or more polynucleotide molecules that encodes N, P and L proteins and an RNA polymerase elongation factor which is an M2 (ORF1) protein of RSV, thereby producing an infectious RSV particle.

2. The method of claim 1, wherein the RSV genome or antigenome and the N, P, L and RNA polymerase elongation factor proteins are expressed by the same expression vector.

3. The method of claim 1, wherein the expression vector encoding the RSV genome or antigenome and the expression vector encoding the N. P. L and RNA polymerase elongation factor proteins are different.

4. The method of claim 1, wherein the N, P, L and RNA polymerase elongation factor proteins are encoded on two or more different expression vectors.

5. The method of claim 4, wherein the N, P, L and RNA polymerase elongation factor proteins are each encoded on different expression vectors.

6. The method of claim 1, wherein the polynucleotide molecule that encodes a RSV genome or antigenome is cDNA.

7. The method of claim 1, wherein the infectious RSV particle is a virus.

8. The method of claim 7, wherein the polynucleotide molecule encoding a RSV genome or antigenome is from a human, bovine or murine RSV sequence.

9. The method of claim 8, wherein the polynucleotide molecule encoding a RSV genome or antigenome is a chimera of a human RSV strain sequence and at least one non-human RSV sequence.

10. The method of claim 1, wherein the polynucleotide molecule encoding the RSV genome or antigenome encodes the sequence of a wild-type RSV strain.

11. The method of claim 1, wherein the polynucleotide molecule encodes a genome or antigenome of a non-human RSV.

12. The method of claim 1, wherein the phenotypic alteration results in attenuation, temperature-sensitivity or host range restriction.

13. The method of claim 1, wherein the polynucleotide encodes a genome or antigenome of a non-human RSV virus or is a chimera of a non-human RSV and at least one other RSV of human or non-human origin.

14. The method of claim 1, wherein he polynucleotide molecule encodes an RSV genome or antigenome of a RSV human vaccine strain that has been modified by a nucleotide insertion, deletion or substitution.

15. The method of claims 14, wherein the modification encodes a phenotypic alteration.

16. The method of claim 15, wherein the phenotypic alteration results in attenuation, temperature-sensitivity or host range restriction.

17. The method of claim 15, wherein the phenotypic alteration is a change in an immunogenic epitope of RSV.

18. The method of claim 1, wherein the polynucleotide molecule that encodes an RSV genome or antigenome of an RSV strain has been modified by inserting a nucleotide sequence that encodes a cytokine or a T-helper epitope.

19. The method of claim 1, wherein the polynucleotide molecule that encodes an RSV genome or antigenome of an RSV strain has been modified by inserting a nucleotide sequence encoding a restriction site marker.

20. The method of claims 1, wherein the polynucleotide molecule that encodes an RSV genome or antigenome of an RSV strain has been modified by inserting a nucleotide sequence encoding a G protein of an RSV subgroup different from that of said RSV strain.

21. The method of claim 1, wherein the polynucleotide molecule that encodes an RSV genome or antigenome of an RSV strain has been modified by inserting a nucleotide sequence encoding a protein of a microbial pathogen capable of eliciting a protective immune response.

22. The method of claim 1, wherein at least one of the viral proteins is supplied by coinfection with RSV.

23. The method of any one of claims 1 to 22, wherein the expression vector which comprises a polynucleotide molecule encoding a RSV genome or antigenome comprises a T7 promoter operably linked to said polynucleotide molecule and the expression vector which comprises one or more polynucleotide molecules that encodes N, P and L proteins and an RNA polymerase elongation factor which is an M2(ORF1) protein of RSV comprises a T7 promoter operably linked to said one or more polynucleotide molecules.

24. A cell or cell-free lysate containing:
(a) an expression vector which comprises a polynucleotide molecule encoding a RSV genome or antigenome; and
(b) an expression vector which comprises one or more polynucleotide molecules that encodes N, P and L proteins and an RNA polymerase elongation factor which is an M2 (ORF1) protein of RSV;
whereby upon expression said RSV genome or antigenome and N, P, L and RNA polymerase elongation factor proteins combine to produce an infectious RSV particle;
and wherein the recombinant RSV genome or antigenome is recombinantly modified by the introduction of a mutation by insertion, rearrangement, deletion or substitution of one or more nucleotides, said mutation producing a phenotype of attenuation, temperature sensitivity, tissue culture adaption, host range restriction or an enhanced immunogenicity of the RSV particle compared to wild-type RSV virus.

25. The cell or lysate of claim 24, wherein the RSV genome or antigenome and the N, P, L and RNA polymerase elongation factor proteins are encoded by the same expression vector.

26. The cell or lysate of claim 24, wherein the expression vector encoding the RSV genome or antigenome and the expression vector encoding the N, P, L and RNA polymerase elongation factor proteins are different.

27. The cell or lysate of claim 24, wherein the N, P, L and RNA polymerase elongation factor proteins are encoded on two or more expression vectors.

28. The cell or lysate of claim 27, wherein the N, P, L and RNA polymerase elongation factor proteins are each encoded on different expression vectors.

29. The cell or lysate of claim 24, wherein the infectious RSV particle is a virus.

30. The cell or lysate of claim 24, wherein the polynucleotide molecule encoding a RSV genome or antigenome is a human, bovine or murine RSV sequence.

31. The cell or lysate of any one of claims 24 to 30, wherein the expression vector which comprises one or more polynucleotide molecules that N, P and L proteins and an RNA polymerase elongation factor which is an M2(ORF1) protein of RSV comprises a T7 promoter operably linked to said one or more polynucleotide molecules.

32. A system for recombinant production of respiratory syncytial virus (RSV) particles comprising one or more DNA polynucleotide molecules, the molecules comprising a polynucleotide encoding a RSV genome or antigenome, a polynucleotide encoding a nucleocapsid (N) protein, a polynucleotide encoding nucleocapsid phosphorprotein (P), a polynucleotide encoding a large polymerase protein (L) and a polynucleotide encoding a RNA polymerase elongation factor protein that is an M2(ORF1) protein or RSV, in which upon expression of said N, P, L and M2 ORF1 proteins, an infectious, self-replicating RSV particle comprising a polynucleotide encoding a RSV genome or antigenome, and said N, P, L and M2 (ORF1) proteins is produced.

33. The system of claim 32, wherein the polynucleotide sequence encoding an RSV genome or antigenome is a human RSV sequence.

34. The system of claim 33, wherein the polynucleotide sequence encoding a human RSV genome or antigenome is SEQ ID NO: 1.

35. The system of claim 32, wherein the polynucleotide encodes a genome or antigenome of a non-human RSV virus or is a chimera of a non-human RSV and at least one other RSV of human or non-human origin.

36. The system of any one of claims 32 to 35, wherein the transcriptional promoter is a T7 promoter.

37. A system for recombinant production of respiratory syncytial virus (RSV) particles comprising one or more expression vectors that comprise a polynucleotide encoding a RSV genome or antigenome, a polynucleotide encoding a nucleocapsid (N) protein, a polynucleotide encoding a nucleocapsid phosphoprotein (P), a polynucleotide encoding a large polymerase protein (L) and a polynucleotide encoding a RNA polymerase elongation factor protein that is an M2(ORF1) protein of RSV, in which upon expression of said N, P, L and M2 ORF1 proteins, an infectious, self-replicating RSV particle comprising a polynucleotide encoding a RSV genome or antigenome, and said N, P, L and M2 (ORF1) proteins is produced.

38. The system of claim 37, in which the RSV genome or antigenome is modified by the insertion, rearrangement, deletion or substitution of one or more nucleotides, said mutation producing a phenotype of attenuation, temperature sensitivity, tissue culture adaptation, host range restriction, or enhanced immunogenicity of the RSV particle compared to wild-type RSV.

39. The system of claim 37 or 38 in which the RSV is a human RSV.

40. The system of claim 37 or 38 in which the RSV genome or antigenome is a chimera of two or more different RSV genomes.

41. The system of claim 37 or 38 in which the chimeric genome or antigenome comprises nucleotide sequences from human and bovine RSV.

42. The system of any of claims 37 to 41 in which a subviral particle is produced.

43. The system of any one of claims 32 to 42, wherein the one or more expression vectors comprises a T7 promoter operably linked to said polynucleotides.

## Patentansprüche

1. Verfahren für das Produzieren eines infektiösen RSV-Partikels ausgehend von einem oder mehreren Polynukleotid-Molekülen, die RSV kodieren, wobei in einer Zelle oder einem zellfreien Lysat ein Expressionsvektor, welcher ein Polynukleotidmolekül enthält, welches ein RSV-Genom oder -Antigenom kodiert, wobei das rekombinante RSV-Genom oder -Antigenom rekombinant durch die Herbeiführung einer Mutation mittels Insertion, Neuanordnung, Deletion oder Substitution von einem oder mehreren Nukleotiden modifiziert worden ist, wobei die Mutation dem RSV-Partikel im Vergleich zu einem Wildtyp RSV einen Phänotyp verleiht, der sich durch eine abgeschwächte Virulenz, Temperaturempfindlichkeit, Gewebekulturanpassung, Beschränkung des Wirtsspektrums oder eine erhöhte Immunogenität auszeichnet, und ein Expressionsvektor, der eine oder mehrere Polynukleotidmoleküle enthält, die N-,P- und L-Proteine sowie einen RNA-Polymerase-Elongationsfaktor, welcher ein M2 (ORF1) Protein von RSV ist, kodieren, ko-exprimiert werden und damit ein infektiöses RSV-Partikel erzeugt wird.

2. Verfahren nach Anspruch 1, wobei das RSV-Genom oder -Antigenom und die N-,P- und L-Proteine sowie das RNA-Polymerase-Elongationsfaktor-Protein durch denselben Expressionsvektor exprimiert werden.

3. Verfahren nach Anspruch 1, wobei der das RSV-Genom oder -Antigenom kodierende Expressionsvektor und der die N-, P- und L-Proteine und das RNA-Polymerase-Elongationsfaktor-Protein kodierende Expressionsvektor unterschiedlich sind.

4. Verfahren nach Anspruch 1, wobei die N-, P- und L-Proteine und das RNA-Polymerase-Elongationsfaktor-Protein auf zwei oder mehr unterschiedlichen Expressionsvektoren kodiert werden.

5. Verfahren nach Anspruch 4, wobei die N-, P- und L-Proteine und das RNA-Polymerase-Elongationsfaktor-Protein jeweils auf einem unterschiedlichen Expressionsvektor kodiert werden.

6. Verfahren nach Anspruch 1, wobei das Polynukleotidmolekül, welches ein RSV-Genom oder -Antigenom kodiert, cDNA ist.

7. Verfahren nach Anspruch 1, wobei das infektiöse RSV-Partikel ein Virus ist.

8. Verfahren nach Anspruch 7, wobei das Polynukleotidmolekül, welches ein RSV-Genom oder -Antigenom kodiert, eine menschliche, bovine oder murine RSV-Sequenz ist.

9. Verfahren nach Anspruch 8, wobei das Polynukleotidmolekül, welches ein RSV-Genom oder -Antigenom kodiert, eine Schimäre einer menschlichen RSV-Stamm-Sequenz und mindestens einer nicht-menschlichen RSV-Sequenz ist.

10. Verfahren nach Anspruch 1, wobei das Polynukleotidmolekül, welches ein RSV-Genom oder -Antigenom kodiert, die Sequenz eines Wildtyp-RSV-Stammes ist.

11. Verfahren nach Anspruch 1, wobei das Polynukleotidmolekül das Genom oder Antigenom eines nicht-menschlichen RSV kodiert.

12. Verfahren nach Anspruch 1, wobei die phänotypische Veränderung eine abgeschwächte Virulenz, Temperaturempfindlichkeit oder Beschränkung des Wirtsspektrums zur Folge hat.

13. Verfahren nach Anspruch 1, wobei das Polynukleotidmolekül das Genom oder Antigenom eines nicht-menschlichen RSV kodiert oder eine Schimäre ist, die aus einer nicht-menschlichen RSV-Sequenz und mindestens einer weiteren RSV-Sequenz menschlichen oder nicht-menschlichen Ursprungs besteht.

14. Verfahren nach Anspruch 1, wobei das Polynukleotidmolekül das Genom oder Antigenom eines menschlichen RSV-Impfstammes kodiert, welcher durch eine Nukleotid-Insertion, -Deletion oder -Substitution modifiziert worden ist.

15. Verfahren nach Anspruch 14, wobei die Modifikation eine phänotypische Veränderung kodiert.

16. Verfahren nach Anspruch 15, wobei die phänotypische Veränderung eine abgeschwächte Virulenz, Temperaturempfindlichkeit oder Beschränkung des Wirtsspektrums zur Folge hat.

17. Verfahren nach Anspruch 15, wobei die phänotypische Veränderung eine Veränderung des immunogenen Epitopes von RSV ist.

18. Verfahren nach Anspruch 1, wobei das Polynukleotidmolekül, welches das RSV-Genom oder -Antigenom eines RSV-Stammes kodiert, durch die Einfügung einer Nukleotidsequenz, welche ein Zytokin oder ein T-Helferzell-Epitop kodiert, modifiziert worden ist.

19. Verfahren nach Anspruch 1, wobei das Polynukleotidmolekül, welches das RSV-Genom oder -Antigenom eines RSV-Stammes kodiert, durch die Einfügung einer Nukleotidsequenz, welche eine Restriktionsenzymerkennungssequenz kodiert, modifiziert worden ist.

20. Verfahren nach Anspruch 1, wobei das Polynukleotidmolekül, welches das RSV-Genom oder -Antigenom eines RSV-Stammes kodiert, durch die Einfügung einer Nukleotidsequenz modifiziert worden ist, welche ein G-Protein einer RSV-Untergruppe kodiert, welche sich von derjenigen des besagten RSV-Stammes unterscheidet.

21. Verfahren nach Anspruch 1, wobei das Polynukleotidmolekül, welches das RSV-Genom oder -Antigenom eines RSV-Stammes kodiert, durch die Einfügung einer Nukleotidsequenz, welche das Protein eines mikrobiellen Pathogens kodiert, welches in der Lage ist eine Schutz erzeugende Immunantwort auszulösen, modifiziert worden ist.

22. Verfahren nach Anspruch 1, wobei mindestens eines der viralen Proteine durch Ko-Infektion mit RSV bereit gestellt wird.

23. Verfahren nach einem der Ansprüche 1 bis 22, wobei der Expressionsvektor, welcher ein Polynukleotidmolekül enthält, welches ein RSV-Genom oder - Antigenom kodiert, einen T7- Promotor enthält, welcher funktionell mit besagtem Polynukleotid verbunden ist und wobei der Expressionsvektor, welcher eines oder mehrere Polynukleotidmoleküle enthält, die N- , P- und L-Proteine sowie einen RNA-Polymerase-Elongationsfaktor, welcher ein M2 (ORF1) Protein von RSV ist, kodieren, einen T7- Promotor enthält, welcher funktionell mit besagten einem oder mehreren Polynukleotidmolekülen verbunden ist.

24. Zelle oder zellfreies Lysat, enthaltend:
(a) einen Expressionsvektor, welcher ein Polynukleotidmolekül enthält, welches ein RSV-Genom oder -Antigenom kodiert; und
(b) einen Expressionsvektor, welcher eines oder mehrere Polynukleotidmoleküle enthält, die N- ,P- und L-Proteine sowie einen RNA-Polymerase-Elongationsfaktor, welcher ein M2 (ORF1) Protein von RSV ist, kodieren;
wobei bei Expression besagtes RSV-Genom oder -Antigenom und die N-, P- und L-Proteine sowie das RNA-Polymerase-Elongationsfaktors-Protein sich kombinieren, sodass ein infektiöses RSV-Partikel produziert wird; und wobei das rekombinante RSV-Genom oder -Antigenom rekombinant durch die Herbeiführung einer Mutation mittels Insertion, Neuanordnung, Deletion oder Substitution von einem oder mehreren Nukleotiden modifiziert worden ist, wobei besagte Mutation dem RSV-Partikel im Vergleich zu einem Wildtyp RSV-Stamm einen Phänotyp verleiht, der sich durch eine abgeschwächte Virulenz, Temperaturempfindlichkeit, Gewebekulturanpassung, Beschränkung des Wirtsspektrums oder eine erhöhte Immunogenität auszeichnet.

25. Zelle oder Lysat nach Anspruch 24, wobei das RSV-Genom oder -Antigenom und die N-, P-, und L-Proteine sowie das RNA-Polymerase-Elongationsfaktor-Protein auf demselben Expressionsvektor kodiert sind.

26. Zelle oder Lysat nach Anspruch 24, wobei der Expressionsvektor, welcher das RSV-Genom oder -Antigenom kodiert, und der Expressionsvektor, welcher die N-, P-, und L-Proteine sowie das RNA-Polymerase-Elongationsfaktor-Protein kodiert, unterschiedlich sind.

27. Zelle oder Lysat nach Anspruch 24, wobei die N-, P-, und L-Proteine und das RNA-Polymerase-Elongationsfaktor-Protein auf zwei oder mehr Expressionsvektoren kodiert sind.

28. Zelle oder Lysat nach Anspruch 27, wobei die N-, P-, und L-Proteine und das RNA-Polymerase-Elongationsfaktor-Protein jeweils auf unterschiedlichen Expressionsvektoren kodiert sind.

29. Zelle oder Lysat nach Anspruch 24, wobei das infektiöse RSV-Partikel ein Virus ist.

30. Zelle oder Lysat nach Anspruch 24, wobei das Polynukleotidmolekül, welches ein RSV-Genom oder -Antigenom kodiert, eine menschliche, bovine oder murine RSV-Sequenz ist.

31. Zelle oder Lysat nach einem der Ansprüche 24 bis 30, wobei der Expressionsvektor, welcher eines oder mehrere Polynukleotidmoleküle enthält, die N- ,P- und L-Proteine sowie einen RNA-Polymerase-Elongationsfaktor, welcher ein M2 (ORF1) Protein von RSV ist, kodieren, einen T7- Promotor enthält, welcher funktionell mit besagtem einem oder mehreren Polynukleotidmolekülen verbunden ist.

32. System zur rekombinanten Produktion von Respiratory-Syncytial-Virus-(RSV)-Partikeln, welche eine oder mehrere DNA-Polynukleotidmoleküle enthalten, wobei diese Moleküle ein Polynukleotid enthalten, welches ein RSV-Genom oder -Antigenom kodiert, ein Polynukleotid, welches ein Nukleocapsid-(N)-Protein kodiert, ein Polynukleotid, welches ein Nukleocapsid-Phosphoprotein (P) kodiert, ein Polynukleotid, welches ein Large-Polymerase-Protein (L) kodiert und ein Polynukleotid, welches ein RNA-Polymerase-Elongations-Faktor-Protein, welches ein M2 (ORF1) Protein von RSV ist, kodiert, wodurch nach Expression der besagten N- ,P-, L- und M2 (ORF1)-Proteine ein infektiöses, zur Selbstreplikation fähiges RSV-Partikel erzeugt wird, welches ein Polynukleotid, welches ein RSV-Genom oder -Antigenom kodiert, und besagte N- ,P-, L- und M2 (ORF1)-Proteine enthält.

33. System nach Anspruch 32, wobei die Polynukleotidsequenz, welche das RSV-Genom oder -Antigenom kodiert, eine menschliche RSV-Sequenz ist.

34. System nach Anspruch 33, wobei die Polynukleotidsequenz, welche das menschliche RSV-Genom oder -Antigenom kodiert, SEQ ID NO:1 ist.

35. System nach Anspruch 32, wobei die Polynukleotidsequenz ein Genom oder Antigenom eines nicht-menschlichen RSV kodiert oder eine Schimäre ist aus einem nicht-menschlichen RSV und mindestens einem weiteren RSV, welcher menschlichen oder nicht-menschlichen Ursprungs ist.

36. System nach einem der Ansprüche 32 bis 35, wobei der transkriptionelle Promotor ein T7-Promotor ist.

37. System zur rekombinanten Produktion von Respiratory-Syncytial-Virus (RSV)-Partikeln, welche einen oder mehrere Expressionsvektoren enthalten, welche ein Polynukleotid enthalten, welches ein RSV-Genom oder -Antigenom kodiert, ein Polynukleotid, welches ein Nukleocapsid-(N)-Protein kodiert, ein Polynukleotid, welches ein Nukleocapsid-Phosphoprotein (P) kodiert, ein Polynukleotid, welches ein Large-Polymerase-Protein (L) kodiert und ein Polynukleotid, welches ein RNA-Polymerase-Elongations-Faktor-Protein, welches ein M2 (ORF1) Protein von RSV ist, kodiert, wodurch nach Expression der besagten N- ,P-, L- und M2 (ORF1)-Proteine ein infektiöses, zur Selbstreplikation fähiges RSV- Partikel erzeugt wird, welches ein Polynukleotid, welches ein RSV-Genom oder - Antigenom kodiert, und besagte N- ,P-, L- und M2 (ORF1)-Proteine enthält,.

38. System nach Anspruch 37, wobei das RSV-Genom oder -Antigenom durch Insertion, Neuanordnung, Deletion oder Substitution von einem oder mehreren Nukleotiden modifiziert worden ist, wobei besagte Mutation dem RSV-Partikel im Vergleich zu einem Wildtyp-RSV einen Phänotyp verleiht, der sich durch eine abgeschwächte Virulenz, Temperaturempfindlichkeit, Gewebekulturanpassung, Beschränkung des Wirtsspektrums oder eine erhöhte Immunogenität auszeichnet.

39. System nach Anspruch 37 oder 38, wobei das RSV menschliches RSV ist.

40. System nach Anspruch 37 oder 38, wobei das RSV-Genom oder -Antigenom eine Schimäre aus zwei oder mehr verschiedenen RSV-Genomen ist.

41. System nach Anspruch 37 oder 38, wobei das RSV-Genom oder -Antigenom eine Schimäre ist, die menschliche und bovine RSV-Sequenzen enthält.

42. System nach einem der Ansprüche 37 bis 41, wobei ein subvirales Partikel erzeugt wird.

43. System nach einem der Ansprüche 32 bis 42, wobei der eine oder mehrere Expressionsvektor(en) einen T7-Promotor enthält/enthalten, welcher funktionell mit besagten Polynukleotiden verbunden ist.

## Revendications

1. Procédé de production d'une particule infectieuse de virus RSV (virus respiratoire syncytial) à partir d'une ou de plusieurs molécule(s) de polynucléotide(s) codant ledit virus RSV, lequel procédé comporte une étape de co-expression, dans une cellule ou dans un lysat acellulaire :
- d'un vecteur d'expression qui comprend une molécule de polynucléotide codant un génome ou un antigénome de virus RSV, lequel génome ou antigénome de virus RSV est modifié par introduction, par recombinaison, d'une mutation par insertion, réarrangement, délétion ou substitution d'un ou de plusieurs nucléotide(s), laquelle mutation produit, au niveau du phénotype, un caractère d'atténuation, de sensibilité à la température, d'adaptation à la culture de tissus ou de restriction de la gamme d'hôtes ou une immunogénicité accrue de la particule de virus RSV par rapport au virus RSV de type sauvage,
- et d'un vecteur d'expression qui comprend une ou plusieurs molécule(s) de polynucléotide(s) codant les protéines N, P et L et un facteur d'élongation d'ARN polymérase qui est une protéine M2(ORF1) de virus RSV,
ce qui conduit à la production d'une particule infectieuse de virus RSV.

2. Procédé conforme à la revendication 1, dans lequel le génome ou l'antigénome de virus RSV, les protéines N, P et L et le facteur d'élongation d'ARN polymérase sont exprimés par le même vecteur d'expression.

3. Procédé conforme à la revendication 1, dans lequel le vecteur d'expression codant le génome ou l'antigénome de virus RSV et le vecteur d'expression codant les protéines N, P et L et le facteur d'élongation d'ARN polymérase sont différents.

4. Procédé conforme à la revendication 1, dans lequel les protéines N, P et L et le facteur d'élongation d'ARN polymérase sont codés dans deux vecteurs d'expression différents ou plus.

5. Procédé conforme à la revendication 4, dans lequel les protéines N, P et L et le facteur d'élongation d'ARN polymérase sont codés, chacun individuellement, dans des vecteurs d'expression différents.

6. Procédé conforme à la revendication 1, dans lequel la molécule de polynucléotide qui code un génome ou un antigénome de virus RSV est un ADNc.

7. Procédé conforme à la revendication 1, dans lequel la particule infectieuse de virus RSV est un virus.

8. Procédé conforme à la revendication 7, dans lequel la molécule de polynucléotide qui code un génome ou un antigénome de virus RSV est issue d'une séquence de virus RSV humain, bovin ou murin.

9. Procédé conforme à la revendication 8, dans lequel la molécule de polynucléotide qui code un génome ou un antigénome de virus RSV est une chimère formée d'une séquence d'une souche de virus RSV humain et d'au moins une séquence de virus RSV non-humain.

10. Procédé conforme à la revendication 1, dans lequel la molécule de polynucléotide qui code un génome ou un antigénome de virus RSV code la séquence d'une souche de virus RSV de type sauvage.

11. Procédé conforme à la revendication 1, dans lequel la molécule de polynucléotide code un génome ou un antigénome d'un virus RSV non-humain.

12. Procédé conforme à la revendication 1, dans lequel l'altération du phénotype consiste en une atténuation, une sensibilité à la température ou une restriction de la gamme d'hôtes.

13. Procédé conforme à la revendication 1, dans lequel le polynucléotide code un génome ou un antigénome d'un virus RSV non-humain ou est une chimère issue d'un virus RSV non-humain et d'au moins un autre virus RSV humain ou non-humain.

14. Procédé conforme à la revendication 1, dans lequel la molécule de polynucléotide code un génome ou un antigénome d'une souche vaccinale humaine de virus RSV, obtenue par modification par insertion, délétion ou substitution de nucléotide(s).

15. Procédé conforme à la revendication 14, dans lequel la modification enraîne une altération du phénotype.

16. Procédé conforme à la revendication 15, dans lequel l'altération du phénotype consiste en une atténuation, une sensibilité à la température ou une restriction de la gamme d'hôtes.

17. Procédé conforme à la revendication 15, dans lequel l'altération du phénotype consiste en un changement au niveau d'un épitope immunogène du virus RSV.

18. Procédé conforme à la revendication 1, dans lequel la molécule de polynucléotide qui code un génome ou un antigénome d'une souche de virus RSV a été modifiée par insertion d'une séquence de nucléotides qui code une cytokine ou un épitope T auxiliaire.

19. Procédé conforme à la revendication 1, dans lequel la molécule de polynucléotide qui code un génome ou un antigénome d'une souche de virus RSV a été modifiée par insertion d'une séquence de nucléotides qui code un marqueur de site de restriction.

20. Procédé conforme à la revendication 1, dans lequel la molécule de polynucléotide qui code un génome ou un antigénome d'une souche de virus RSV a été modifiée par insertion d'une séquence de nucléotides qui code une protéine G d'un sous-groupe de virus RSV différent de celui de ladite souche de virus RSV.

21. Procédé conforme à la revendication 1, dans lequel la molécule de polynucléotide qui code un génome ou un antigénome d'une souche de virus RSV a été modifiée par insertion d'une séquence de nucléotides qui code une protéine d'un agent pathogène microbien capable de provoquer une réponse immune protectrice.

22. Procédé conforme à la revendication 1, dans lequel au moins l'une des protéines virales est apportée par co-infection par un virus RSV.

23. Procédé conforme à l'une des revendications 1 à 22, dans lequel le vecteur d'expression qui comprend une molécule de polynucléotide codant un génome ou un antigénome de virus RSV comprend un promoteur T7 opérationnellement lié à ladite molécule de polynucléotide, et le vecteur d'expression qui comprend une ou plusieurs molécule(s) de polynucléotide(s) codant les protéines N, P et L et un facteur d'élongation d'ARN polymérase qui est une protéine M2(ORF1) de virus RSV comprend un promoteur T7 opérationnellement lié à ladite ou auxdites molécule(s) de polynucléotide(s).

24. Cellule ou lysat acellulaire, contenant :
a) un vecteur d'expression qui comprend une molécule de polynucléotide codant un génome ou un antigénome de virus RSV ;
b) et un vecteur d'expression qui comprend une ou plusieurs molécule(s) de polynucléotide(s) codant les protéines N, P et L et un facteur d'élongation d'ARN polymérase qui est une protéine M2(ORF1) de virus RSV ;
dans laquelle ou lequel, après expression, ledit génome ou antigénome de virus RSV, lesdites protéines N, P et L et ledit facteur d'élongation d'ARN polymérase se combinent pour produire une particule infectieuse de virus RSV,
et dans laquelle ou lequel le génome ou antigénome de virus RSV est modifié par introduction, par recombinaison, d'une mutation par insertion, réarrangement, délétion ou substitution d'un ou de plusieurs nucléotide(s), laquelle mutation produit, au niveau du phénotype, un caractère d'atténuation, de sensibilité à la température, d'adaptation à la culture de tissus ou de restriction de la gamme d'hôtes ou une immunogénicité accrue de la particule de virus RSV par rapport auvirus RSV de type sauvage.

25. Cellule ou lysat acellulaire, conforme à la revendication 24, dans laquelle ou lequel le génome ou l'antigénome de virus RSV, les protéines N, P et L et le facteur d'élongation d'ARN polymérase sont codés par le même vecteur d'expression.

26. Cellule ou lysat acellulaire, conforme à la revendication 24, dans laquelle ou lequel le vecteur d'expression codant le génome ou l'antigénome de virus RSV et le vecteur d'expression codant les protéines N, P et L et le facteur d'élongation d'ARN polymérase sont différents.

27. Cellule ou lysat acellulaire, conforme à la revendication 24, dans laquelle ou lequel les protéines N, P et L et le facteur d'élongation d'ARN polymérase sont codées dans deux vecteurs d'expression différents ou plus.

28. Cellule ou lysat acellulaire, conforme à la revendication 27, dans laquelle ou lequel les protéines N, P et L et le facteur d'élongation d'ARN polymérase sont codés, chacun individuellement, dans des vecteurs d'expression différents.

29. Cellule ou lysat acellulaire, conforme à la revendication 24, dans laquelle ou lequel la particule infectieuse de virus RSV est un virus.

30. Cellule ou lysat acellulaire, conforme à la revendication 24, dans laquelle ou lequel la molécule de polynucléotide qui code un génome ou un antigénome de virus RSV est une séquence de virus RSV humain, bovin ou murin.

31. Cellule ou lysat acellulaire, conforme à l'une des revendications 24 à 30, dans laquelle ou lequel le vecteur d'expression qui comprend une ou plusieurs molécule(s) de polynucléotide(s) codant les protéines N, P et L et un facteur d'élongation d'ARN polymérase qui est une protéine M2(ORF1) de virus RSV comprend un promoteur T7 opérationnellement lié à ladite ou auxdites molécule(s) de polynucléotide(s).

32. Système de production par recombinaison de particules de virus syncytial respiratoire (virus RSV), qui comporte une ou plusieurs molécule(s) de polynucléotide(s) ADN, lesquelles molécules comprennent :
- un polynucléotide codant un génome ou un antigénome de virus RSV,
- un polynucléotide codant une protéine de nucléocapside (protéine N),
- un polynucléotide codant une phosphoprotéine de nucléocapside (protéine P),
- un polynuclétide codant une protéine grande sous-unité de polymérase (protéine L),
- et un polynucléotide codant un facteur d'élongation d'ARN polymérase qui est une protéine M2(ORF1) de virus RSV,
et dans lequel, après expression desdites protéines N, P, L et M2(ORF1), est produite une particule infectieuse autoréplicative de virus RSV qui comprend lesdites protéines N, P, L et M2(ORF1) et un polynucléotide qui code un génome ou un antigénome de virus RSV.

33. Système conforme à la revendication 32, dans lequel la séquence du polynucléotide codant un génome ou un antigénome de virus RSV est une séquence de virus RSV humain.

34. Système conforme à la revendication 33, dans lequel la séquence du polynucléotide codant un génome ou un antigénome de virus RSV humain est la séquence présentée en tant que Séquence n° 1.

35. Système conforme à la revendication 32, dans lequel le polynucléotide code un génome ou un antigénome d'un virus RSV non-humain ou est une chimère d'un virus RSV non-humain et d'au moins un autre virus RSV d'origine humaine ou non-humaine.

36. Système conforme à l'une des revendications 32 à 35, dans lequel le promoteur de transcription est un promoteur T7.

37. Système de production par recombinaison de particules de virus syncytial respiratoire (virus RSV), qui comprend un ou plusieurs vecteur(s) d'expression comportant :
- un polynucléotide codant un génome ou un antigénome de virus RSV,
- un polynucléotide codant une protéine de nucléocapside (protéine N),
- un polynucléotide codant une phosphoprotéine de nucléocapside (protéine P),
- un polynuclétide codant une protéine grande sous-unité de polymérase (protéine L),
- et un polynucléotide codant un facteur d'élongation d'ARN polymérase qui est une protéine M2(ORF1) de virus RSV,
et dans lequel, après expression desdites protéines N, P, L et M2(ORF1), est produite une particule infectieuse autoréplicative de virus RSV qui comprend lesdites protéines N, P, L et M2(ORF1) et un polynucléotide, qui code un génome ou un antigénome de virus RSV.

38. Système conforme à la revendication 37, dans lequel le génome ou l'antigénome de virus RSV est modifié par insertion, réarrangement, délétion ou substitution d'un ou de plusieurs nucléotide(s), mutation qui produit, au niveau du phénotype, un caractère d'atténuation, de sensibilité à la température, d'adaptation à la culture de tissus ou de restriction de la gamme d'hôtes ou une immunogénicité accrue de la particule de virus RSV par rapport à celle du virus RSV de type sauvage.

39. Système conforme à la revendication 37 ou 38, dans lequel le virus RSV est un virus RSV humain.

40. Système conforme à la revendication 37 ou 38, dans lequel le génome ou l'antigénome de virus RSV est une chimère formée de deux génomes de virus RSV différents ou plus.

41. Système conforme à la revendication 40, dans lequel le génome ou l'antigénome chimérique comporte des séquences de nucléotides provenant d'un virus RSV humain et d'un virus RSV bovin.

42. Système conforme à l'une des revendications 37 à 41, dans lequel c'est une particule sous-virale qui est produite.

43. Système conforme à l'une des revendications 37 à 42, dans lequel le ou les vecteur(s) d'expression comprend ou comprennent un promoteur T7, opérationnellement lié auxdits polynucléotides.


### SEQUENCE LISTING

(1) GENERAL INFORMATION:
   (i) APPLICANT:
      (A) NAME: The Government of the United States of America, as represented by the Secretary of the Department of Health and Human Services
      (B) STREET: Box OTT
      (C) CITY: Bethesda
      (D) STATE: Maryland
      (E) COUNTRY: United States of America
      (F) POSTAL CODE (ZIP): 20892
      (G) TELEPHONE: (301) 496-7056
      (H) TELEFAX: (301) 402-0220
      (I) TELEX:
   (ii) TITLE OF INVENTION: PRODUCTION OF INFECTIOUS RESPIRATORY SYNCYTIAL VIRUS FROM CLONED NUCLEOTIDE SEQUENCES
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (v) CURRENT APPLICATION DATA:
      (A) APPLICATION NUMBER: WO
      (B) FILING DATE:
      (C) CLASSIFICATION:
   (vi) PRIOR APPLICATION DATA:
      (A) APPLICATION NUMBER: US 60/007,083
      (B) FILING DATE: 27-SEP-1995
   (vii) ATTORNEY/AGENT INFORMATION:
      (A) NAME: Parmelee, Steven W.
      (B) REGISTRATION NUMBER: 31,990
      (C) REFERENCE/DOCKET NUMBER: 15280-250-1PC
   (viii) TELECOMMUNICATION INFORMATION:
      (A) TELEPHONE: 206-467-9600
      (B) TELEFAX: 415-576-0300
(2) INFORMATION FOR SEQ ID NO:1:
   (i) SEQUENCE CHARACTERISTICS:
      (A) LENGTH: 15223 base pairs
      (B) TYPE: nucleic acid
      (C) STRANDEDNESS: single
      (D) TOPOLOGY: linear
   (ii) MOLECULE TYPE: other
   (xi) SEQUENCE DESCRIPTION: SEQ ID NO:1:
   ACGSGAAAAA ATGCGTACAA CAAACTTGCA TAAACCAAAA AAATGGGGCA AATAAGAATT 60
   TGATAAGTAC CACTTAAATT TAACTCCCTT GGTTAGAGAT GGGCAGCAAT TCATTGAGTA 120
   TGATAAAAGT TAGATTACAA AATTTGTTTG ACAATGATGA AGTAGCATTG TTAAAAATAA   180
   CATGCTATAC TGATAAATTA ATACATTTAA CTAATGCTTT GGCTAAGGCA GTGATACATA   240
   CAATCAAATT GAATGGCATT GTGTTTGTGC ATGTTATTAC AAGTAGTGAT ATTTGCCCTA   300
   ATAATAATAT TGTAGTAAAA TCCAATTTCA CAACAATGCC AGTACTACAA AATGGAGGTT   360
   ATATATGGGA AATGATGGAA TTAACACATT GCTCTCAACC TAATGGTCTA CTAGATGACA   420
   ATTGTGAAAT TAAATTCTCC AAAAAACTAA GTGATTCAAC AATGACCAAT TATATGAATC   480
   AATTATCTGA ATTACTTGGA TTTGATCTTA ATCCATAAAT TATAATTAAT ATCAACTAGC   540
   AAATCAATGT CACTAACACC ATTAGTTAAT ATAAAACTTA ACAGAAGACA AAAATGGGGC   600
   AAATAAATCA ATTCAGCCAA CCCAACCATG GACACAACCC ACAATGATAA TACACCACAA   660
   AGACTGATGA TCACAGACAT GAGACCGTTG TCACTTGAGA CCATAATAAC ATCACTAACC   720
   AGAGACATCA TAACACACAA ATTTATATAC TTGATAAATC ATGAATGCAT AGTGAGAAAA   780
   CTTGATGAAA AGCAGGCCAC ATTTACATTC CTGGTCAACT ATGAAATGAA ACTATTACAC   840
   AAAGTAGGAA GCACTAAATA TAAAAAATAT ACTGAATACA ACACAAAATA TGGCACTTTC   900
   CCTATGCCAA TATTCATCAA TCATGATGGG TTCTTAGAAT GCATTGGCAT TAAGCCTACA   960
   AAGCATACTC CCATAATATA CAAGTATGAT CTCAATCCAT AAATTTCAAC ACAATATTCA  1020
   CACAATCTAA AACAACAACT CTATGCATAA CTATACTCCA TAGTCCAGAT GGAGCCTGAA  1080
   AATTATAGTA ATTTAAAACT TAAGGAGAGA TATAAGATAG AAGATGGGGC AAATACAACC  1140
   ATGGCTCTTA GCAAAGTCAA GTTGAATGAT ACACTCAACA AAGATCAACT TCTGTCATCC  1200
   AGCAAATACA CCATCCAACG GAGCACAGGA GATAGTATTG ATACTCCTAA TTATGATGTG  1260
   CAGAAACACA TCAATAAGTT ATGTGGCATG TTATTAATCA CAGAAGATGC TAATCATAAA  1320
   TTCACTGGGT TAATAGGTAT GTTATATGCG ATGTCTAGGT TAGGAAGAGA AGACACCATA  1380
   AAAATACTCA GAGATGCGGG ATATCATGTA AAAGCAAATG GAGTAGATGT AACAACACAT  1440
   CGTCAAGACA TTAATGGAAA AGAAATGAAA TTTGAAGTGT TAACATTGGC AAGCTTAACA  1500
   ACTGAAATTC AAATCAACAT TGAGATAGAA TCTAGAAAAT CCTACAAAAA AATGCTAAAA  1560
   GAAATGGGAG AGGTAGCTCC AGAATACAGG CATGACTCTC CTGATTGTGG GATGATAATA  1620
   TTATGTATAG CAGCATTAGT AATAACTAAA TTAGCAGCAG GGGACAGATC TGGTCTTACA  1680
   GCCGTGATTA GGAGAGCTAA TAATGTCCTA AAAAATGAAA TGAAACGTTA CAAAGGCTTA  1740
   CTACCCAAGG ACATAGCCAA CAGCTTCTAT GAAGTGTTTG AAAAACATCC CCACTTTATA  1800
   GATGTTTTTG TTCATTTTGG TATAGCACAA TCTTCTACCA GAGGTGGCAG TAGAGTTGAA  1860
   GGGATTTTTG CAGGATTGTT TATGAATGCC TATGGTGCAG GGCAAGTGAT GTTACGGTGG  1920
   GGAGTCTTAG CAAAATCAGT TAAAAATATT ATGTTAGGAC ATGCTAGTGT GCAAGCAGAA  1980
   ATGGAACAAG TTGTTGAGGT TTATGAATAT GCCCAAAAAT TGGGTGGTGA AGCAGGATTC  2040
   TACCATATAT TGAACAACCC AAAAGCATCA TTATTATCTT TGACTCAATT TCCTCACTTC  2100
   TCCAGTGTAG TATTAGGCAA TGCTGCTGGC CTAGGCATAA TGGGAGAGTA CAGAGGTACA  2160
   CCGAGGAATC AAGATCTATA TGATGCAGCA AAGGCATATG CTGAACAACT CAAAGAAAAT 2220
   GGTGTGATTA ACTACAGTGT ACTAGACTTG ACAGCAGAAG AACTAGAGGC TATCAAACAT 2280
   CAGCTTAATC CAAAAGATAA TGATGTAGAG CTTTGAGTTA ATAAAAAATG GGGCAAATAA 2340
   ATCATCATGG AAAAGTTTGC TCCTGAATTC CATGGAGAAG ATGCAAACAA CAGGGCTACT 2400
   AAATTCCTAG AATCAATAAA GGGCAAATTC ACATCACCCA AAGATCCCAA GAAAAAAGAT 2460
   AGTATCATAT CTGTCAACTC AATAGATATA GAAGTAACCA AAGAAAGCCC TATAACATCA 2520
   AATTCAACTA TTATCAACCC AACAAATGAG ACAGATGATA CTGCAGGGAA CAAGCCCAAT 2580
   TATCAAAGAA AACCTCTAGT AAGTTTCAAA GAAGACCCTA CACCAAGTGA TAATCCCTTT 2640
   TCTAAACTAT ACAAAGAAAC CATAGAAACA TTTGATAACA ATGAAGAAGA ATCCAGCTAT 2700
   TCATACGAAG AAATAAATGA TCAGACAAAC GATAATATAA CAGCAAGATT AGATAGGATT 2760
   GATGAAAAAT TAAGTGAAAT ACTAGGAATG CTTCACACAT TAGTAGTGGC AAGTGCAGGA 2820
   CCTACATCTG CTCGGGATGG TATAAGAGAT GCCATGGTTG GTTTAAGAGA AGAAATGATA 2880
   GAAAAAATCA GAACTGAAGC ATTAATGACC AATGACAGAT TAGAAGCTAT GGCAAGACTC 2940
   AGGAATGAGG AAAGTGAAAA GATGGCAAAA GACACATCAG ATGAAGTGTC TCTCAATCCA 3000
   ACATCAGAGA AATTGAACAA CCTATTGGAA GGGAATGATA GTGACAATGA TCTATCACTT 3060
   GAAGATTTCT GATTAGTTAC CAATCTTCAC ATCAACACAC AATACCAACA GAAGACCAAC 3120
   AAACTAACCA ACCCAATCAT CCAACCAAAC ATCCATCCGC CAATCAGCCA AACAGCCAAC 3180
   AAAACAACCA GCCAATCCAA AACTAACCAC CCGGAAAAAA TCTATAATAT AGTTACAAAA 3240
   AAAGGAAAGG GTGGGGCAAA TATGGAAACA TACGTGAACA AGCTTCACGA AGGCTCCACA 3300
   TACACAGCTG CTGTTCAATA CAATGTCTTA GAAAAAGACG ATGACCCTGC ATCACTTACA 3360
   ATATGGGTGC CCATGTTCCA ATCATCTATG CCAGCAGATT TACTTATAAA AGAACTAGCT 3420
   AATGTCAACA TACTAGTGAA ACAAATATCC ACACCCAAGG GACCTTCACT AAGAGTCATG 3480
   ATAAACTCAA GAAGTGCAGT GCTAGCACAA ATGCCCAGCA AATTTACCAT ATGCGCTAAT 3540
   GTGTCCTTGG ATGAAAGAAG CAAACTAGCA TATGATGTAA CCACACCCTG TGAAATCAAG 3600
   GCATGTAGTC TAACATGCCT AAAATCAAAA AATATGTTGA CTACAGTTAA AGATCTCACT 3660
   ATGAAGACAC TCAACCCTAC ACATGATATT ATTGCTTTAT GTGAATTTGA AAACATAGTA 3720
   ACATCAAAAA AAGTCATAAT ACCAACATAC CTAAGATCCA TCAGTGTCAG AAATAAAGAT 3780
   CTGAACACAC TTGAAAATAT AACAACCACT GAATTCAAAA ATGCTATCAC AAATGCAAAA 3840
   ATCATCCCTT ACTCAGGATT ACTATTAGTC ATCACAGTGA CTGACAACAA AGGAGCATTC 3900
   AAATACATAA AGCCACAAAG TCAATTCATA GTAGATCTTG GAGCTTACCT AGAAAAAGAA 3960
   AGTATATATT ATGTTACCAC AAATTGGAAG CACACAGCTA CACGATTTGC AATCAAACCC 4020
   ATGGAAGATT AACCTTTTTC CTCTACATCA GTGTGTTAAT TCATACAAAC TTTCTACCTA 4080
   CATTCTTCAC TTCACCATCA CAATCACAAA CACTCTGTGG TTCAACCAAT CAAACAAAAC 4140
   TTATCTGAAG TCCCAGATCA TCCCAAGTCA TTGTTTATCA GATCTAGTAC TCAAATAAGT 4200
   TAATAAAAAA TATACACATG GGGCAAATAA TCATTGGAGG AAATCCAACT AATCACAATA 4260
   TCTGTTAACA TAGACAAGTC CACACACCAT ACAGAATCAA CCAATGGAAA ATACATCCAT 4320
   AACAATAGAA TTCTCAAGCA AATTCTGGCC TTACTTTACA CTAATACACA TGATCACAAC 4380
   AATAATCTCT TTGCTAATCA TAATCTCCAT CATGATTGCA ATACTAAACA AACTTTGTGA 4440
   ATATAACGTA TTCCATAACA AAACCTTTGA GTTACCAAGA GCTCGAGTCA ACACATAGCA 4500
   TTCATCAATC CAACAGCCCA AAACAGTAAC CTTGCATTTA AAAATGAACA ACCCCTACCT 4560
   CTTTACAACA CCTCATTAAC ATCCCACCAT GCAAACCACT ATCCATACTA TAAAGTAGTT 4620
   AATTAAAAAT AGTCATAACA ATGAACTAGG ATATCAAGAC TAACAATAAC ATTGGGGCAA 4680
   ATGCAAACAT GTCCAAAAAC AAGGACCAAC GCACCGCTAA GACATTAGAA AGGACCTGGG 4740
   ACACTCTCAA TCATTTATTA TTCATATCAT CGTGCTTATA TAAGTTAAAT CTTAAATCTG 4800
   TAGCACAAAT CACATTATCC ATTCTGGCAA TGATAATCTC AACTTCACTT ATAATTGCAG 4860
   CCATCATATT CATAGCCTCG GCAAACCACA AAGTCACACC AACAACTGCA ATCATACAAG 4920
   ATGCAACAAG CCAGATCAAG AACACAACCC CAACATACCT CACCCAGAAT CCTCAGCTTG 4980
   GAATCAGTCC CTCTAATCCG TCTGAAATTA CATCACAAAT CACCACCATA CTAGCTTCAA 5040
   CAACACCAGG AGTCAAGTCA ACCCTGCAAT CCACAACAGT CAAGACCAAA AACACAACAA 5100
   CAACTCAAAC ACAACCCAGC AAGCCCACCA CAAAACAACG CCAAAACAAA CCACCAAGCA 5160
   AACCCAATAA TGATTTTCAC TTTGAAGTGT TCAACTTTGT ACCCTGCAGC ATATGCAGCA 5220
   ACAATCCAAC CTGCTGGGCT ATCTGCAAAA GAATACCAAA CAAAAAACCA GGAAAGAAAA 5280
   CCACTACCAA GCCCACAAAA AAACCAACCC TCAAGACAAC CAAAAAAGAT CCCAAACCTC 5340
   AAACCACTAA ATCAAAGGAA GTACCCACCA CCAAGCCCAC AGAAGAGCCA ACCATCAACA 5400
   CCACCAAAAC AAACATCATA ACTACACTAC TCACCTCCAA CACCACAGGA AATCCAGAAC 5460
   TCACAAGTCA AATGGAAACC TTCCACTCAA CTTCCTCCGA AGGCAATCCA AGCCCTTCTC 5520
   AAGTCTCTAC AACATCCGAG TACCCATCAC AACCTTCATC TCCACCCAAC ACACCACGCC 5580
   AGTAGTTACT TAAAAACATA TTATCACAAA AGGCCTTGAC CAACTTAAAC AGAATCAAAA 5640
   TAAACTCTGG GGCAAATAAC AATGGAGTTG CTAATCCTCA AAGCAAATGC AATTACCACA 5700
   ATCCTCACTG CAGTCACATT TTGTTTTGCT TCTGGTCAAA ACATCACTGA AGAATTTTAT 5760
   CAATCAACAT GCAGTGCAGT TAGCAAAGGC TATCTTAGTG CTCTGAGAAC TGGTTGGTAT 5820
   ACCAGTGTTA TAACTATAGA ATTAAGTAAT ATCAAGAAAA ATAAGTGTAA TGGAACAGAT 5880
   GCTAAGGTAA AATTGATAAA ACAAGAATTA GATAAATATA AAAATGCTGT AACAGAATTG 5940
   CAGTTGCTCA TGCAAAGCAC ACAAGCAACA AACAATCGAG CCAGAAGAGA ACTACCAAGG 6000
   TTTATGAATT ATACACTCAA CAATGCCAAA AAAACCAATG TAACATTAAG CAAGAAAAGG 6060
   AAAAGAAGAT TTCTTGGTTT TTTGTTAGGT GTTGGATCTG CAATCGCCAG TGGCGTTGCT 6120
   GTATCTAAGG TCCTGCACCT AGAAGGGGAA GTGAACAAGA TCAAAAGTGC TCTACTATCC 6180
   ACAAACAAGG CTGTAGTCAG CTTATCAAAT GGAGTTAGTG TTTTAACCAG CAAAGTGTTA 6240
   GACCTCAAAA ACTATATAGA TAAACAATTG TTACCTATTG TGAACAAGCA AAGCTGCAGC 6300
   ATATCAAATA TAGAAACTGT GATAGAGTTC CAACAAAAGA ACAACAGACT ACTAGAGATT 6360
   ACCAGGGAAT TTAGTGTTAA TGCAGGCGTA ACTACACCTG TAAGCACTTA CATGTTAACT 6420
   AATAGTGAAT TATTGTCATT AATCAATGAT ATGCCTATAA CAAATGATCA GAAAAAGTTA 6480
   ATGTCCAACA ATGTTCAAAT AGTTAGACAG CAAAGTTACT CTATCATGTC CATAATAAAA 6540
   GAGGAAGTCT TAGCATATGT AGTACAATTA CCACTATATG GTGTTATAGA TACACCCTGT 6600
   TGGAAACTAC ACACATCCCC TCTATGTACA ACCAACACAA AAGAAGGGTC CAACATCTGT 6660
   TTAACAAGAA CTGACAGAGG ATGGTACTGT GACAATGCAG GATCAGTATC TTTCTTCCCA 6720
   CAAGCTGAAA CATGTAAAGT TCAATCAAAT CGAGTATTTT GTGACACAAT GAACAGTTTA 6780
   ACATTACCAA GTGAAGTAAA TCTCTGCAAT GTTGACATAT TCAACCCCAA ATATGATTGT 6840
   AAAATTATGA CTTCAAAAAC AGATGTAAGC AGCTCCGTTA TCACATCTCT AGGAGCCATT 6900
   GTGTCATGCT ATGGCAAAAC TAAATGTACA GCATCCAATA AAAATCGTGG AATCATAAAG 6960
   ACATTTTCTA ACGGGTGCGA TTATGTATCA AATAAAGGGG TGGACACTGT GTCTGTAGGT 7020
   AACACATTAT ATTATGTAAA TAAGCAAGAA GGTAAAAGTC TCTATGTAAA AGGTGAACCA 7080
   ATAATAAATT TCTATGACCC ATTAGTATTC CCCTCTGATG AATTTGATGC ATCAATATCT 7140
   CAAGTCAACG AGAAGATTAA CCAGAGCCTA GCATTTATTC GTAAATCCGA TGAATTATTA 7200
   CATAATGTAA ATGCTGGTAA ATCCACCACA AATATCATGA TAACTACTAT AATTATAGTG 7260
   ATTATAGTAA TATTGTTATC ATTAATTGCT GTTGGACTGC TCTTATACTG TAAGGCCAGA 7320
   AGCACACCAG TCACACTAAG CAAAGATCAA CTGAGTGGTA TAAATAATAT TGCATTTAGT 7380
   AACTAAATAA AAATAGCACC TAATCATGTT CTTACAATGG TTTACTATCT GCTCATAGAC 7440
   AACCCATCTG TCATTGGATT TTCTTAAAAT CTGAACTTCA TCGAAACTCT CATCTATAAA 7500
   CCATCTCACT TACACTATTT AAGTAGATTC CTAGTTTATA GTTATATAAA ACACAATTGC 7560
   ATGCCAGATT AACTTACCAT CTGTAAAAAT GAAAACTGGG GCAAATATGT CACGAAGGAA 7620
   TCCTTGCAAA TTTGAAATTC GAGGTCATTG CTTAAATGGT AAGAGGTGTC ATTTTAGTCA 7680
   TAATTATTTT GAATGGCCAC CCCATGCACT GCTTGTAAGA CAAAACTTTA TGTTAAACAG 7740
   AATACTTAAG TCTATGGATA AAAGTATAGA TACCTTATCA GAAATAAGTG GAGCTGCAGA 7800
   GTTGGACAGA ACAGAAGAGT ATGCTCTTGG TGTAGTTGGA GTGCTAGAGA GTTATATAGG 7860
   ATCAATAAAC AATATAACTA AACAATCAGC ATGTGTTGCC ATGAGCAAAC TCCTCACTGA 7920
   ACTCAATAGT GATGATATCA AAAAGCTGAG GGACAATGAA GAGCTAAATT CACCCAAGAT 7980
   AAGAGTGTAC AATACTGTCA TATCATATAT TGAAAGCAAC AGGAAAAACA ATAAACAAAC 8040
   TATCCATCTG TTAAAAAGAT TGCCAGCAGA CGTATTGAAG AAAACCATCA AAAACACATT 8100
   GGATATCCAT AAGAGCATAA CCATCAACAA CCCAAAAGAA TCAACTGTTA GTGATACAAA 8160
   TGACCATGCC AAAAATAATG ATACTACCTG ACAAATATCC TTGTAGTATA ACTTCCATAC 8220
   TAATAACAAG TAGATGTAGA GTTACTATGT ATAATCAAAA GAACACACTA TATTTCAATC 8280
   AAAACAACCC AAATAACCAT ATGTACTCAC CGAATCAAAC ATTCAATGAA ATCCATTGGA 8340
   CCTCTCAAGA ATTGATTGAC ACAATTCAAA ATTTTCTACA ACATCTAGGT ATTATTGAGG 8400
   ATATATATAC AATATATATA TTAGTGTCAT AACACTCAAT TCTAACACTC ACCACATCGT 8460
   TACATTATTA ATTCAAACAA TTCAAGTTGT GGGACAAAAT GGATCCCATT ATTAATGGAA 8520
   ATTCTGCTAA TGTTTATCTA ACCGATAGTT ATTTAAAAGG TGTTATCTCT TTCTCAGAGT 8580
   GTAATGCTTT AGGAAGTTAC ATATTCAATG GTCCTTATCT CAAAAATGAT TATACCAACT 8640
   TAATTAGTAG ACAAAATCCA TTAATAGAAC ACATGAATCT AAAGAAACTA AATATAACAC 8700
   AGTCCTTAAT ATCTAAGTAT CATAAAGGTG AAATAAAATT AGAAGAACCT ACTTATTTTC 8760
   AGTCATTACT TATGACATAC AAGAGTATGA CCTCGTCAGA ACAGATTGCT ACCACTAATT 8820
   TACTTAAAAA GATAATAAGA AGAGCTATAG AAATAAGTGA TGTCAAAGTC TATGCTATAT 8880
   TGAATAAACT AGGGCTTAAA GAAAAGGACA AGATTAAATC CAACAATGGA CAAGATGAAG 8940
   ACAACTCAGT TATTACGACC ATAATCAAAG ATGATATACT TTCAGCTGTT AAAGATAATC 9000
   AATCTCATCT TAAAGCAGAC AAAAATCACT CTACAAAACA AAAAGACACA ATCAAAACAA 9060
   CACTCTTGAA GAAATTGATG TGTTCAATGC AACATCCTCC ATCATGGTTA ATACATTGGT 9120
   TTAACTTATA CACAAAATTA AACAACATAT TAACACAGTA TCGATCAAAT GAGGTAAAAA 9180
   ACCATGGGTT TACATTGATA GATAATCAAA CTCTTAGTGG ATTTCAATTT ATTTTGAACC 9240
   AATATGGTTG TATAGTTTAT CATAAGGAAC TCAAAAGAAT TACTGTGACA ACCTATAATC 9300
   AATTCTTGAC ATGGAAAGAT ATTAGCCTTA GTAGATTAAA TGTTTGTTTA ATTACATGGA 9360
   TTAGTAACTG CTTGAACACA TTAAATAAAA GCTTAGGCTT AAGATGCGGA TTCAATAATG 9420
   TTATCTTGAC ACAACTATTC CTTTATGGAG ATTGTATACT AAAGCTATTT CACAATGAGG 9480
   GGTTCTACAT AATAAAAGAG GTAGAGGGAT TTATTATGTC TCTAATTTTA AATATAACAG 9540
   AAGAAGATCA ATTCAGAAAA CGATTTTATA ATAGTATGCT CAACAACATC ACAGATGCTG 9600
   CTAATAAAGC TCAGAAAAAT CTGCTATCAA GAGTATGTCA TACATTATTA GATAAGACAG 9660
   TGTCCGATAA TATAATAAAT GGCAGATGGA TAATTCTATT AAGTAAGTTC CTTAAATTAA 9720
   TTAAGCTTGC AGGTGACAAT AACCTTAACA ATCTGAGTGA ACTATATTTT TTGTTCAGAA 9780
   TATTTGGACA CCCAATGGTA GATGAAAGAC AAGCCATGGA TGCTGTTAAA ATTAATTGCA 9840
   ATGAGACCAA ATTTTACTTG TTAAGCAGTC TGAGTATGTT AAGAGGTGCC TTTATATATA 9900
   GAATTATAAA AGGGTTTGTA AATAATTACA ACAGATGGCC TACTTTAAGA AATGCTATTG 9960
   TTTTACCCTT AAGATGGTTA ACTTACTATA AACTAAACAC TTATCCTTCT TTGTTGGAAC 10020
   TTACAGAAAG AGATTTGATT GTGTTATCAG GACTACGTTT CTATCGTGAG TTTCGGTTGC 10080
   CTAAAAAAGT GGATCTTGAA ATGATTATAA ATGATAAAGC TATATCACCT CCTAAAAATT 10140
   TGATATGGAC TAGTTTCCCT AGAAATTACA TGCCATCACA CATACAAAAC TATATAGAAC 10200
   ATGAAAAATT AAAATTTTCC GAGAGTGATA AATCAAGAAG AGTATTAGAG TATTATTTAA 10260
   GAGATAACAA ATTCAATGAA TGTGATTTAT ACAACTGTGT AGTTAATCAA AGTTATCTCA 10320
   ACAACCCTAA TCATGTGGTA TCATTGACAG GCAAAGAAAG AGAACTCAGT GTAGGTAGAA 10380
   TGTTTGCAAT GCAACCGGGA ATGTTCAGAC AGGTTCAAAT ATTGGCAGAG AAAATGATAG 10440
   CTGAAAACAT TTTACAATTC TTTCCTGAAA GTCTTACAAG ATATGGTGAT CTAGAACTAC 10500
   AAAAAATATT AGAACTGAAA GCAGGAATAA GTAACAAATC AAATCGCTAC AATGATAATT 10560
   ACAACAATTA CATTAGTAAG TGCTCTATCA TCACAGATCT CAGCAAATTC AATCAAGCAT 10620
   TTCGATATGA AACGTCATGT ATTTGTAGTG ATGTGCTGGA TGAACTGCAT GGTGTACAAT 10680
   CTCTATTTTC CTGGTTACAT TTAACTATTC CTCATGTCAC AATAATATGC ACATATAGGC 10740
   ATGCACCCCC CTATATAGGA GATCATATTG TAGATCTTAA CAATGTAGAT GAACAAAGTG 10800
   GATTATATAG ATATCACATG GGTGGCATCG AAGGGTGGTG TCAAAAACTA TGGACCATAG 10860
   AAGCTATATC ACTATTGGAT CTAATATCTC TCAAAGGGAA ATTCTCAATT ACTGCTTTAA 10920
   TTAATGGTGA CAATCAATCA ATAGATATAA GCAAACCAAT CAGACTCATG GAAGGTCAAA 10980
   CTCATGCTCA AGCAGATTAT TTGCTAGCAT TAAATAGCCT TAAATTACTG TATAAAGAGT 11040
   ATGCAGGCAT AGGCCACAAA TTAAAAGGAA CTGAGACTTA TATATCACGA GATATGCAAT 11100
   TTATGAGTAA AACAATTCAA CATAACGGTG TATATTACCC AGCTAGTATA AAGAAAGTCC 11160
   TAAGAGTGGG ACCGTGGATA AACACTATAC TTGATGATTT CAAAGTGAGT CTAGAATCTA 11220
   TAGGTAGTTT GACACAAGAA TTAGAATATA GAGGTGAAAG TCTATTATGC AGTTTAATAT 11280
   TTAGAAATGT ATGGTTATAT AATCAGATTG CTCTACAATT AAAAAATCAT GCATTATGTA 11340
   ACAATAAACT ATATTTGGAC ATATTAAAGG TTCTGAAACA CTTAAAAACC TTTTTTAATC 11400
   TTGATAATAT TGATACAGCA TTAACATTGT ATATGAATTT ACCCATGTTA TTTGGTGGTG 11460
   GTGATCCCAA CTTGTTATAT CGAAGTTTCT ATAGAAGAAC TCCTGACTTC CTCACAGAGG 11520
   CTATAGTTCA CTCTGTGTTC ATACTTAGTT ATTATACAAA CCATGACTTA AAAGATAAAC 11580
   TTCAAGATCT GTCAGATGAT AGATTGAATA AGTTCTTAAC ATGCATAATC ACGTTTGACA 11640
   AAAACCCTAA TGCTGAATTC GTAACATTGA TGAGAGATCC TCAAGCTTTA GGGTCTGAGA 11700
   GACAAGCTAA AATTACTAGC GAAATCAATA GACTGGCAGT TACAGAGGTT TTGAGTACAG 11760
   CTCCAAACAA AATATTCTCC AAAAGTGCAC AACATTATAC TACTACAGAG ATAGATCTAA 11820
   ATGATATTAT GCAAAATATA GAACCTACAT ATCCTCATGG GCTAAGAGTT GTTTATGAAA 11880
   GTTTACCCTT TTATAAAGCA GAGAAAATAG TAAATCTTAT ATCAGGTACA AAATCTATAA 11940
   CTAACATACT GGAAAAAACT TCTGCCATAG ACTTAACAGA TATTGATAGA GCCACTGAGA 12000
   TGATGAGGAA AAACATAACT TTGCTTATAA GGATACTTCC ATTGGATTGT AACAGAGATA 12060
   AAAGAGAGAT ATTGAGTATG GAAAACCTAA GTATTACTGA ATTAAGCAAA TATGTTAGGG 12120
   AAAGATCTTG GTCTTTATCC AATATAGTTG GTGTTACATC ACCCAGTATC ATGTATACAA 12180
   TGGACATCAA ATATACTACA AGCACTATAT CTAGTGGCAT AATTATAGAG AAATATAATG 12240
   TTAACAGTTT AACACGTGGT GAGAGAGGAC CCACTAAACC ATGGGTTGGT TCATCTACAC 12300
   AAGAGAAAAA AACAATGCCA GTTTATAATA GACAAGTCTT AACCAAAAAA CAGAGAGATC 12360
   AAATAGATCT ATTAGCAAAA TTGGATTGGG TGTATGCATC TATAGATAAC AAGGATGAAT 12420
   TCATGGAAGA ACTCAGCATA GGAACCCTTG GGTTAACATA TGAAAAGGCC AAGAAATTAT 12480
   TTCCACAATA TTTAAGTGTC AATTATTTGC ATCGCCTTAC AGTCAGTAGT AGACCATGTG 12540
   AATTCCCTGC ATCAATACCA GCTTATAGAA CAACAAATTA TCACTTTGAC ACTAGCCCTA 12600
   TTAATCGCAT ATTAACAGAA AAGTATGGTG ATGAAGATAT TGACATAGTA TTCCAAAACT 12660
   GTATAAGCTT TGGCCTTAGT TTAATGTCAG TAGTAGAACA ATTTACTAAT GTATGTCCTA 12720
   ACAGAATTAT TCTCATACCT AAGCTTAATG AGATACATTT GATGAAACCT CCCATATTCA 12780
   CAGGTGATGT TGATATTCAC AAGTTAAAAC AAGTGATACA AAAACAGCAT ATGTTTTTAC 12840
   CAGACAAAAT AAGTTTGACT CAATATGTGG AATTATTCTT AAGTAATAAA ACACTCAAAT 12900
   CTGGATCTCA TGTTAATTCT AATTTAATAT TGGCACATAA AATATCTGAC TATTTTCATA 12960
   ATACTTACAT TTTAAGTACT AATTTAGCTG GACATTGGAT TCTGATTATA CAACTTATGA 13020
   AAGATTCTAA AGGTATTTTT GAAAAAGATT GGGGAGAGGG ATATATAACT GATCATATGT 13080
   TTATTAATTT GAAAGTTTTC TTCAATGCTT ATAAGACCTA TCTCTTGTGT TTTCATAAAG 13140
   GTTATGGCAA AGCAAAGCTG GAGTGTGATA TGAACACTTC AGATCTTCTA TGTGTATTGG 13200
   AATTAATAGA CAGTAGTTAT TGGAAGTCTA TGTCTAAGGT ATTTTTAGAA CAAAAAGTTA 13260
   TCAAATACAT TCTTAGCCAA GATGCAAGTT TACATAGAGT AAAAGGATGT CATAGCTTCA 13320
   AATTATGGTT TCTTAAACGT CTTAATGTAG CAGAATTCAC AGTTTGCCCT TGGGTTGTTA 13380
   ACATAGATTA TCATCCAACA CATATGAAAG CAATATTAAC TTATATAGAT CTTGTTAGAA 13440
   TGGGATTGAT AAATATAGAT AGAATACACA TTAAAAATAA ACACAAATTC AATGATGAAT 13500
   TTTATACTTC TAATCTCTTC TACATTAATT ATAACTTCTC AGATAATACT CATCTATTAA 13560
   CTAAACATAT AAGGATTGCT AATTCTGAAT TAGAAAATAA TTACAACAAA TTATATCATC 13620
   CTACACCAGA AACCCTAGAG AATATACTAG CCAATCCGAT TAAAAGTAAT GACAAAAAGA 13680
   CACTGAATGA CTATTGTATA GGTAAAAATG TTGACTCAAT AATGTTACCA TTGTTATCTA 13740
   ATAAGAAGCT TATTAAATCG TCTGCAATGA TTAGAACCAA TTACAGCAAA CAAGATTTGT 13800
   ATAATTTATT CCCTATGGTT GTGATTGATA GAATTATAGA TCATTCAGGC AATACAGCCA 13860
   AATCCAACCA ACTTTACACT ACTACTTCCC ACCAAATATC CTTAGTGCAC AATAGCACAT 13920
   CACTTTACTG CATGCTTCCT TGGCATCATA TTAATAGATT CAATTTTGTA TTTAGTTCTA 13980
   CAGGTTGTAA AATTAGTATA GAGTATATTT TAAAAGATCT TAAAATTAAA GATCCCAATT 14040
   GTATAGCATT CATAGGTGAA GGAGCAGGGA ATTTATTATT GCGTACAGTA GTGGAACTTC 14100
   ATCCTGACAT AAGATATATT TACAGAAGTC TGAAAGATTG CAATGATCAT AGTTTACCTA 14160
   TTGAGTTTTT AAGGCTGTAC AATGGACATA TCAACATTGA TTATGGTGAA AATTTGACCA 14220
   TTCCTGCTAC AGATGCAACC AACAACATTC ATTGGTCTTA TTTACATATA AAGTTTGCTG 14280
   AACCTATCAG TCTTTTTGTC TGTGATGCCG AATTGTCTGT AACAGTCAAC TGGAGTAAAA 14340
   TTATAATAGA ATGGAGCAAG CATGTAAGAA AGTGCAAGTA CTGTTCCTCA GTTAATAAAT 14400
   GTATGTTAAT AGTAAAATAT CATGCTCAAG ATGATATTGA TTTCAAATTA GACAATATAA   14460
   CTATATTAAA AACTTATGTA TGCTTAGGCA GTAAGTTAAA GGGATCGGAG GTTTACTTAG   14520
   TCCTTACAAT AGGTCCTGCG AATATATTCC CAGTATTTAA TGTAGTACAA AATGCTAAAT   14580
   TGATACTATC AAGAACCAAA AATTTCATCA TGCCTAAGAA AGCTGATAAA GAGTCTATTG   14640
   ATGCAAATAT TAAAAGTTTG ATACCCTTTC TTTGTTACCC TATAACAAAA AAAGGAATTA   14700
   ATACTGCATT GTCAAAACTA AAGAGTGTTG TTAGTGGAGA TATACTATCA TATTCTATAG   14760
   CTGGACGTAA TGAAGTTTTC AGCAATAAAC TTATAAATCA TAAGCATATG AACATCTTAA   14820
   AATGGTTCAA TCATGTTTTA AATTTCAGAT CAACAGAACT AAACTATAAC CATTTATATA   14880
   TGGTAGAATC TACATATCCT TACCTAAGTG AATTGTTAAA CAGCTTGACA ACCAATGAAC   14940
   TTAAAAAACT GATTAAAATC ACAGGTAGTC TGTTATACAA CTTTCATAAT GAATAATGAA   15000
   TAAAGATCTT ATAATAAAAA TTCCCATAGC TATACACTAA CACTGTATTC AATTATAGTT   15060
   ATTAAAAATT AAAAATCATA TAATTTTTTA AATAACTTTT AGTGAACTAA TCCTAAAGTT   15120
   ATCATTTTAA TCTTGGAGGA ATAAATTTAA ACCCTAATCT AATTGGTTTA TATGTGTATT   15180
   AACTAAATTA CGAGATATTA GTTTTTGACA CTTTTTTTCT CGT                    15223